(19) Europäisches Patentamt
European Patent Office
Office européen des brevets

(11) EP 4 684 742 A1

(12) # EUROPEAN PATENT APPLICATION
published in accordance with Art. 153(4) EPC

(43) Date of publication:
**28.01.2026 Bulletin 2026/05**

(21) Application number: **24863086.5**

(22) Date of filing: **22.08.2024**

(51) International Patent Classification (IPC):
***A61B 17/06*** $^{(2006.01)}$ ***A61B 17/34*** $^{(2006.01)}$
***A61F 2/00*** $^{(2006.01)}$ ***A61B 17/00*** $^{(2006.01)}$

(52) Cooperative Patent Classification (CPC):
**A61B 17/00; A61B 17/06; A61B 17/34; A61F 2/00**

(86) International application number:
**PCT/KR2024/012552**

(87) International publication number:
**WO 2025/053505 (13.03.2025 Gazette 2025/11)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **07.09.2023 KR 20230118890**
**10.07.2024 KR 20240090893**

(71) Applicant: **Neo Dr. Inc.**
**Wonju-si, Gangwon-do 26311 (KR)**

(72) Inventors:
- **KIM, Hyeongjun**
  **Wonju-si Gangwon-do 26461 (KR)**
- **KIM, Jiyoo**
  **Wonju-si Gangwon-do 26461 (KR)**

(74) Representative: **Caspary, Karsten et al**
**Kroher-Strobel**
**Rechts- und Patentanwälte PartmbB**
**Bavariaring 20**
**80336 München (DE)**

(54) **LIFTING THREAD, METHOD FOR MANUFACTURING LIFTING THREAD, AND LIFTING THREAD INSERTION DEVICE**

(57) The present invention relates to a lifting thread, a method for manufacturing the lifting thread, and a lifting thread insertion device. The lifting thread is manufactured in a coil spring shape by twisting a medical thread, and then winding (coiling) same around the outer peripheral surface (circumference) of a needle, such that when the lifting thread is tensioned and compressed during a skin lifting procedure, a first elastic force according to the torsional stress of the medical thread, and the greater stress obtained by adding the tensile or compressive stress of the lifting thread to the first elastic force, that is, a second elastic force, are applied so as to maintain, as is, the elasticity of the skin without sagging of the lifted skin even after a long period of time has elapsed after the skin lifting procedure, and thus a lifting effect can be maximized.

FIG. 3

10
P



Processed by Luminess, 75001 PARIS (FR)

## Description

### TECHNICAL FIELD

The present disclosure relates to a lifting thread, a method for manufacturing the lifting thread, and a lifting thread insertion device, and more particularly, to a lifting thread that can maximize a lifting effect by manufacturing a lifting thread in a coil spring shape by twisting a medical thread to generate the torsional stress and winding (coiling) the medical thread around the outer peripheral surface (circumference) of a needle so that a first elastic force according to the torsional stress of the medical thread, and the greater stress obtained by adding the tensile or compressive stress of the lifting thread to the first elastic force, that is, a second elastic force, are applied so as to maintain, as is, the elasticity of the skin without sagging of the lifted skin even after a long period of time has elapsed after the skin lifting procedure, a method for manufacturing the lifting thread, and a lifting thread insertion device.

### BACKGROUND

With the recent improvement in living standards, modern people are paying more attention to maintaining their healthy skin in addition to maintaining their healthy body. Consequently, interest in improving skin beauty and preventing skin aging is on the rise.

Skin aging is manifested by phenomena such as dry skin, slow skin regeneration, and accumulation of aging dead skin cells. In addition, the amount of collagen to be synthesized that supports the skin's epidermis decreases and elastin degenerates, causing wrinkles to appear.

Skin wrinkles are determined by collagen fibers present in the dermis layer. Collagen molecules are produced by fibroblasts, secreted into the dermis layer, and then formed into collagen fibers through auto-association. These collagen fibers are involved in the mechanical strength of the skin, tissue cohesion, cell adhesion, cell differentiation, and the like in the dermal layer of the skin.

Collagen production is reduced or deformation continues to accumulate due to internal and external factors, which causes wrinkles.

Like this, collagen, which is related to skin wrinkles, gradually decreases due to photoaging and intrinsic aging, causing the skin to thin, which becomes the main cause of wrinkle formation.

Typically, there are various methods to remove wrinkles on the skin of the face, lower jaw, neck, and the like such as face lift, implantation of fillers, botox injections, and laser skin regeneration. However, recently, since a procedure method for embedding lifting threads, which are threads attached to a needle (syringe needle), is much simpler than surgery and causes little disruption to daily life, the procedure method is gaining attention as a method for lifting and wrinkle improvement.

The lifting thread used in the skin lifting procedure is an absorbable or non-absorbable thread with a diameter of about 0.2 to 0.8 mm made of materials such as silicone, polypropylene, and polydioxanone, which lifts up the aged skin on the surface and the aged soft tissue (SMAS) inside the skin to remove wrinkles and restore firmness.

Non-surgical wrinkle removal surgery is widely used because it minimizes scarring, which is the biggest disadvantage of scalpel surgery, by using a needle under simple local anesthesia, and not only does it reduce irritation or damage to the treated area, but it also reduces bleeding and swelling caused by the procedure, and it gives natural tension after the procedure, making the skin elastic and younger-looking.

The lifting threads in the related art have scale-like thorns formed on the surface to pull and fix sagging skin tissue to improve wrinkles or lift, but they have disadvantages such as severe pain after the procedure, difficulty opening the mouth, and dimples or uneven marks left on the skin surface.

In order to solve this problem, the European registration publication EP1726317 (registration number 04775207.6 Surgical thread and Cosmetic Surgery method) that is the document in the related art discloses a technique for the skin lifting procedure.

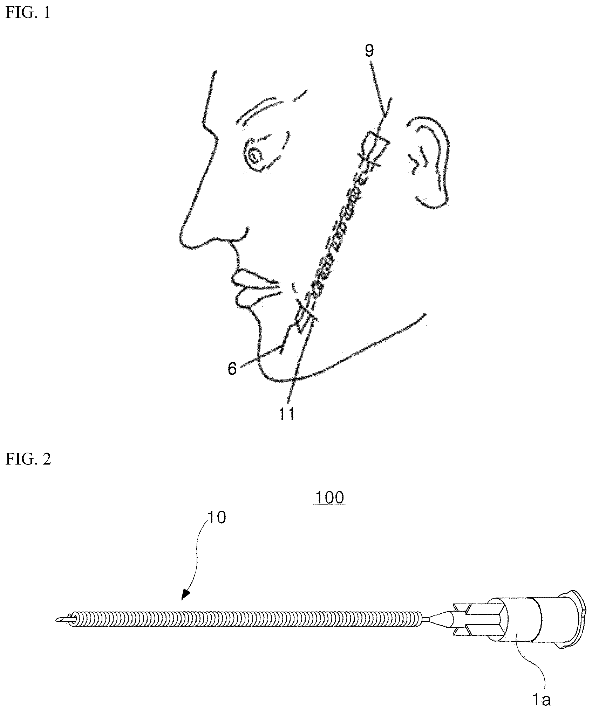

FIG. 1 is a conceptual diagram for describing a lifting thread and a lifting method in the related art disclosed in the document in the related art.

Referring to FIG. 1, a lifting thread 9 in the related art is manufactured by heat-treating a thread wound in a spiral shape (coil spring shape) to have elasticity, and during a lifting procedure, the lifting thread is embedded in a skin layer (skin tissue) 11 using a needle, and then tensioned or compressed to have a predetermined length to have a lifting and wrinkle improvement effect on the skin layer by the elasticity (elastic restoring force) of the lifting thread 9.

However, lifting threads usually go through a heat treatment process to increase elasticity. In the related art, in order to maintain sufficient elasticity, the lifting threads are subjected to a high temperature (a temperature exceeding 75% of the melting temperature, for example, exceeding 85°C in the case of polydioxanone polymers) heat treatment process. However, when the heat treatment temperature of the lifting thread is maintained at a high temperature, the elasticity of the lifting thread is improved, but after the skin lifting procedure, the lifting thread embedded inside the skin layer has a problem in that the time for hydrolysis is too short and it is easily hydrolyzed.

In addition, in skin lifting methods in the related art, it is complicated to insert a plastic surgery thread into the skin layer in an extended state (stretched state) during a skin lifting procedure, and in order to increase the elasticity of the thread to enhance the lifting effect, a thread with a relatively thick outer diameter needs to be used, and in addition, there are disadvantages in that the tensile stress easily disappears depending on the time and degree of stretching when simply coiling and heat treating the thread, and it is difficult to properly control the elasticity of the lifting thread.

SUMMARY

The present disclosure was invented to solve the above-described problem, and a first purpose of the present disclosure is to provide a lifting thread that can maximize a lifting effect by manufacturing the lifting thread in a coil spring shape by twisting a medical thread and then winding (coiling) the medical thread around the outer peripheral surface (circumference) of a needle so that, when the lifting thread is tensioned or compressed during a skin lifting procedure, a first elastic force according to the torsional stress of the medical thread, and the greater stress obtained by adding the tensile or compressive stress of the lifting thread to the first elastic force are applied so as to maintain, as is, the elasticity of the skin without sagging of the lifted skin because a significant portion of the elasticity of the thread remains even after a long period of time has elapsed after the skin lifting procedure, a method for manufacturing the lifting thread, and a lifting thread insertion device.

Also, a second purpose of the present disclosure is to provide a lifting thread that can perform the skin lifting procedure very easily and quickly, a method for manufacturing the lifting thread, and a lifting thread insertion device by providing a withdrawal portion formed as an extension at a rear end portion of the lifting thread so that, when the lifting thread is embedded inside the skin layer, the withdrawal portion is exposed to the outside of the skin layer to have a predetermined length and thus the lifting thread can be easily pulled and tensioned by pulling the withdrawal portion, a method for manufacturing the lifting thread, and a lifting thread insertion device.

Furthermore, a third purpose of the present disclosure is to provide a lifting thread obtained by providing a non-slip combination portion at a rear end portion of the lifting thread so that, when a needle is pulled while the lifting thread is embedded in the surgical location inside the skin layer and the tip of the lifting thread is fixed inside the skin layer, the rear end portion of the lifting thread is pulled together with the needle by the non-slip combination portion such that the lifting thread is stably tensioned, a method for manufacturing the lifting thread, and a lifting thread insertion device.

Moreover, a fourth purpose of the present disclosure is to provide a lifting thread in which a pair of anchors are provided at both end portions of the lifting thread, a center of the anchor of the tip of the lifting thread is blocked to prevent the needle from passing therethrough so that, when a needle is pushed into the skin layer, the needle pushes the tip of the lifting thread into the skin layer, the location of the anchor of the rear end portion of the lifting thread is fixed by being caught at the beginning of the skin layer, and when the needle is pushed into the skin layer, the lifting thread is tensioned and an elastic restoring force is generated, when the needle is removed, the anchor of the tip of the lifting thread is fixed at the insertion location inside the skin layer, and the anchor of the rear end portion of the lifting thread is caught at the beginning of the skin layer so that the tension state of the lifting thread can be maintained, a method for manufacturing the lifting thread, and a lifting thread insertion device.

In order to achieve the above-described purpose, a lifting thread according to an example of the present disclosure is a lifting thread having a coil spring structure in which a twisted thread obtained by twisting one or more medical threads centered on a longitudinal central axis L of the medical thread is wound (coiled), and the elastic modulus of the lifting thread can be adjusted by adjusting a twist angle $\theta$, which is an angle at which the twisted thread is twisted.

A lifting thread 10 according to a first example embodiment of the present disclosure may be wound (coiled) into a tensile coil spring shape using a twisted thread in which one or more medical threads are twisted to have a twist angle $\theta$ centered on the longitudinal central axis L. At this time, the elastic modulus of the lifting thread 10 may be adjusted by adjusting the twist angle $\theta$ as an angle between the longitudinal central axis L (imaginary line) of the medical thread and a twist line C. At this time, the twist line C may mean a line that deforms as a parallel line that appears in a longitudinal direction of a surface of the original, untwisted medical thread is twisted. As the twist angle $\theta$ formed by the longitudinal central axis L and the twist line C of the twisted thread after being formed into the lifting thread 10 increases, the elastic modulus of the lifting thread 10 may increase.

A lifting thread 20 according to a second example embodiment of the present disclosure may be wound (coiled) into a compressed coil spring shape using a twisted thread that is twisted to have a twist angle $\theta$ centered on a longitudinal central axis L of one or more medical threads. At this time, the elastic modulus of the lifting thread 20 may be adjusted by adjusting the twist angle $\theta$ as an angle between the longitudinal central axis L (imaginary line) of the twisted thread and the twist line C. The twist line C may mean a line that deforms by twisting a parallel line that appears in a longitudinal direction of a surface of the original, untwisted medical thread. As the twist angle $\theta$ formed by the longitudinal central axis L and the twist line C of the twisted thread after being formed into the lifting thread 20 increases, the elastic modulus of the lifting thread 20 may increase.

A lifting thread 30 according to a third example embodiment of the present disclosure may include a composite structure in which a first coil spring structure in which a first twisted thread in which a portion of one or more medical threads is twisted is wound (coiled) and a second coil spring structure in which a second twisted thread in which another portion of the medical thread is twisted in the opposite direction is wound (coiled) are connected so that they operate independently. In the lifting thread 30 with the composite structure, the first coil spring structure may be a tensile coil spring structure, and the second coil spring structure may be a compressive coil spring structure. In addition, the first twisted thread and the second twisted thread may be the same twisted thread, or may be threads in which different twisted threads are attached and connected. The above-described first twist angle of the tensile coil spring portion and the above-described second twist angle of the compressive coil spring portion may be the same or different, and may be independently adjusted to adjust the elastic modulus of the composite structure lifting thread 30. That is, the elastic modulus of the lifting thread 30 may be adjusted by adjusting the twist angle $\theta$ formed by the twist line C, which is a line that deforms by twisting a parallel line that appears in a longitudinal direction of a surface of the original medical thread, and the longitudinal central axis L of the corresponding medical thread after being manufactured as a lifting thread of a coil spring structure. Specifically, as the twist angle $\theta$ increases, the elastic coefficient of the lifting thread 30 may increase. More specifically, the twist angle $\theta$ may be the first twist angle and/or the second twist angle, and these may be independently adjusted. In this case, directions indicated by the first twist angle of the tensile coil spring portion and the second twist angle of the compressive coil spring portion may be opposite to each other.

A method for manufacturing a lifting thread according to the first example embodiment of the present disclosure is a method for manufacturing the lifting thread 10 having tensile coil spring characteristics by winding a medical thread around the outer peripheral surface of a needle.

The method for manufacturing the lifting thread 10 according to the first example embodiment of the present disclosure is to manufacture the lifting thread 10 having tensile coil spring characteristics by winding a medical thread 1 around the outer peripheral surface of a needle, and may include a twisted thread forming step S110 of twisting the medical thread in one direction so that the torsional stress is generated inside the medical thread 1; and

> a lifting thread coiling step S120 of forming a lifting thread having tensile coil spring characteristics by winding (coiling) the twisted thread from the tip of the outer peripheral surface of the needle toward a rear end portion thereof in the same direction as a twisting direction of the twisted thread, in which
> during the skin lifting procedure, a first elastic force is applied due to the torsional stress generated by twisting the medical thread in advance, and a second elastic force is applied due to the tensile stress generated by tensioning the lifting thread 10 during the lifting procedure.

A method for manufacturing a lifting thread 20 according to the second example embodiment of the present disclosure is a method for manufacturing a lifting thread having compressive coil spring characteristics by winding a medical thread around the outer peripheral surface of a needle.

The manufacturing method of the lifting thread 20 according to the second example embodiment of the present disclosure may include a twisting forming step S210 of twisting the medical thread in one direction so that the torsional stress is generated inside the medical thread; and a coiling step S220 of the lifting thread 20 of forming a lifting thread having compressive coil spring characteristics by winding (coiling) the twisted thread from the tip of the outer peripheral surface of the needle toward a rear end portion thereof in a direction opposite to a twisting direction of the twisted thread, in which
during the skin lifting procedure, the first elastic force due to the torsional stress generated by twisting the medical thread in advance and the second elastic force, which is the combined compressive stress generated by compressing the lifting thread 20 during the lifting procedure, are applied.

A method for manufacturing a lifting thread 30 according to the third example embodiment of the present disclosure is a method for manufacturing a lifting thread of a composite structure having tensile coil spring characteristics and compressive coil spring characteristics.

The manufacturing method of the lifting thread 30 according to the third example embodiment of the present disclosure may include a first twisted thread forming step S310 of twisting a medical thread in one direction so that the torsional stress is generated inside the medical thread; a first coiling step S320 of winding the first twisted thread into a coil spring shape from the tip of the outer peripheral surface of the needle 1 toward the rear end portion thereof; a second twisted thread forming step S330 of twisting the remaining portion of the medical thread in a direction opposite to the first twisting direction so that the torsional stress is generated in the opposite direction inside the medical thread; and a second coiling step S340 of winding the second twisted thread in the same direction as the first coiling direction while moving toward the rear end portion aroung the outer peripheral surface of the needle 1.

The lifting thread 30 is technically characterized by being a composite structure that includes both tensile coil spring characteristics and compressive coil spring characteristics. For example, when the top of the lifting thread 30 is formed into a compressed coil spring shape in the first coiling step, the rear end of the lifting thread 30 may be formed into a tensile coil spring shape in the second coiling step. In addition, when the tip of the lifting thread 30 is formed into a tensile coil spring shape in the first coiling step, the rear end of the lifting thread 30 may be formed into a compression coil spring shape in the second coiling step.

In addition, the method for manufacturing the lifting thread according to the first, second or third example embodiment of the present disclosure may further include a heat treatment process for improving the elasticity of the medical thread.

Meanwhile, the lifting thread 10 according to the first example embodiment of the present disclosure may include a lifting thread formed to have tensile coil spring characteristics by twisting the medical thread in one direction so that the torsional stress is generated inside the medical thread and winding the medical thread from the tip of the outer peripheral surface of the needle 1 toward the rear end portion thereof in the same direction as the twisting direction of the medical thread, in which, during the skin lifting procedure, the first elastic force due to the torsional stress generated by twisting the medical thread in advance and the second elastic force due to the tensile stress generated by tensioning the lifting thread 10 during the procedure are combined to form the elastic force.

Meanwhile, the lifting thread 20 according to the second example embodiment of the present disclosure may include a lifting thread formed to have compressive coil spring characteristics by twisting the medical thread in one direction so that the torsional stress is generated inside the medical thread and winding the medical thread from the tip of the outer peripheral surface of the needle 1 toward the rear end portion thereof in the direction opposite to the twisting direction of the medical thread, in which, during the skin lifting procedure, a first elastic force is applied due to the torsional stress generated by twisting the medical thread in advance, and a second elastic force is applied due to the compressive stress generated as the lifting thread 20 is compressed during the skin lifting procedure.

Meanwhile, the lifting thread 30 according to the third example embodiment of the present disclosure may include a lifting thread formed by subjecting the medical thread to first twisting in one direction so that the torsional stress is generated inside the medical thread, winding one end of the medical thread in a coil spring shape from the tip of the outer peripheral surface of the needle 1 toward the rear end portion thereof, subjecting it to first coiling, and then subjecting the remaining portion of the medical thread to second twisting in the direction opposite to the first twisting direction so that the torsional stress is generated inside the medical thread in the opposite direction, and subjecting the remaining portion of the medical thread to second winding around the outer peripheral surface of the needle 1 toward the rear end portion thereof in the same direction as the first coiling direction, in which the lifting thread has a composite structure having tensile coil spring characteristics and compressive coil spring characteristics.

Meanwhile, a lifting thread insertion device according to the first example embodiment of the present disclosure includes a needle; and a lifting thread formed by winding a medical thread from the tip of the outer peripheral surface of the needle toward the rear end portion thereof in a tensile coil spring structure in the same direction as a twisting direction of the medical thread while twisting the medical thread in one direction so that the torsional stress is generated in the internal tissue of the medical thread, in which, during the skin lifting procedure, a first elastic force due to the torsional stress generated by twisting the medical thread in advance and a second elastic force due to the tensile stress generated by tensioning the lifting thread 10 during the lifting procedure are combined.

In addition, the lifting thread is characterized in that the elastic modulus of the lifting thread 10 increases as a twist angle θ increases. At this time, the twist angle θ of the lifting thread is an angle formed by the twist line C that deforms as a parallel line that appears in a longitudinal direction around the surface of the untwisted medical thread is twisted around the longitudinal central axis L (imaginary line) of the medical thread and the central axis L.

In addition, the elastic modulus of the lifting thread may be adjusted by increasing or decreasing a thickness of the medical thread, an inner diameter of the lifting thread, a pitch, and the like.

In addition, an anchor is provided at each of the tip and rear end portions of the lifting thread, and when the anchors are pulled in directions facing each other, the anchors are provided with a hook jaw so as to be caught in the skin layer and continuously maintain the tension state of the lifting thread.

Also, the lifting thread insertion device according to the first example embodiment of the present disclosure may further include a sliding holder formed in a pipe shape so as to be slidable back and forth on the outer peripheral surface of the needle, and configured to support a rear end portion of the lifting thread so that, when the needle is pulled out from the skin layer during the skin lifting procedure, the lifting thread does not come out of the skin layer together with the needle but remains embedded in the skin layer; and a withdrawal portion connected to the rear end portion of the lifting thread so as to be exposed to the outside of the skin layer by a predetermined length and to pull and tension the lifting thread when the lifting thread is embedded in the skin layer.

In addition, the lifting thread insertion device 100 according to the first example embodiment of the present disclosure may further include a non-slip combination portion which is formed on the rear end portion of the lifting thread and is relatively tightly coupled to the outer peripheral surface of the needle compared to other portions, and which supports the lifting thread so that it is not pushed backward when the needle is inserted into the skin layer during a lifting procedure, and when the location of the tip of the lifting thread is fixed in the skin layer and the needle is pulled backward, the rear end portion of the lifting thread is pulled out together with the needle and is tensioned to have elastic restoring force. At this time, a sliding holder may be further provided at the rear of the non-slip combination portion, which is formed in a pipe shape and is connected to the outer peripheral surface of the needle so as to be able to slide back and forth, and which may support the rear end portion of the lifting thread when separating the lifting thread from the needle during a lifting procedure and separate the needle from the skin layer (skin tissue).

In addition, the lifting thread insertion device 100 according to the first example embodiment of the present disclosure may include an anchor provided at each of the tip and rear end portions of the lifting thread, and when the anchors are pulled in directions facing each other, each of the anchors may further include a hook jaw so as to be caught in the skin layer and continuously maintain the tension state of the lifting thread.

A lifting thread insertion device 200 according to the second example embodiment of the present disclosure includes a needle; and a lifting thread formed by winding a medical thread in a compressive coil spring structure in a direction opposite to a twisting direction of the medical thread from the tip of the outer peripheral surface of the needle toward the rear end portion thereof while twisting the medical thread in one direction so that the torsional stress is generated inside the medical thread, in which, during the skin lifting procedure, a first elastic force is applied due to the torsional stress generated by twisting the medical thread in advance, and a second elastic force is applied due to the compressive stress generated by compressing the lifting thread 20 during the procedure.

The lifting thread is characterized in that the elastic modulus of the lifting thread 20 increases as the twist angle $\theta$ increases. At this time, the twist angle $\theta$ of the lifting thread is an angle formed by the twist line C that deforms as a parallel line that appears in the longitudinal direction on the surface of the untwisted medical thread is twisted around the longitudinal central axis L (imaginary line) of the medical thread and the central axis L.

In addition, the elastic modulus of the lifting thread 20 may also be adjusted by increasing or decreasing a thickness of the medical thread, an inner diameter of the lifting thread, a pitch, and the like.

The lifting thread insertion device 200 according to the second example embodiment of the present disclosure is formed in a pipe shape and is slidably coupled to the outer peripheral surface (circumference) of the needle, and has a technical feature that further includes a sliding holder that, when the needle is pushed into the skin layer, the lifting thread is not pushed backwards of the needle, but is embedded into the skin layer together with the needle and is compressed inside the skin layer.

A lifting thread insertion device 300 according to the third example embodiment of the present disclosure includes a needle; and a medical thread, which is first twisted in one direction so that the torsional stress is generated inside the medical thread, and then first wound in a tensile coil spring shape around the outer peripheral surface of the needle in the same direction as a twisting direction of the medical thread; and second twisting of the medical thread in the opposite direction so that the torsional stress is generated in the internal tissue of the medical thread in a direction opposite to that of the first torsional stress, and then a compressed coil is wound around the outer peripheral surface of the needle in the same direction as the first twisting direction, in which the lifting thread has tensile coil spring characteristics and compressive coil spring characteristics.

In addition, during the skin lifting procedure, the tension coil spring portion of the lifting thread in the skin layer has a dense pitch and acts as a base or column, and the compression coil spring portion has a non-dense pitch and acts to elastically push up the skin layer.

In addition, the elasticity of the lifting thread according to the torsional stress is configured to be controlled by increasing or decreasing the twist angle of the medical thread.

Meanwhile, the lifting thread insertion device according to the first, second or third example embodiment may configure a multi-stage lifting thread by connecting a plurality of lifting threads in series. A multi-stage lifting thread M may be a plurality of independent lifting threads that are the same or different from each other, connected in series, and specifically, the multi-stage lifting thread M may be a plurality of independent lifting threads that are the same or different from each other, connected in series through n-1 connecting portions (not shown) (wherein n is any positive integer). The multi-stage lifting thread M may have a higher elastic modulus of a lifting thread F located at the front than a lifting thread B located at the rear with respect to the direction of entry of the lifting thread during the lifting procedure. For example, the elastic modulus of the lifting thread F of the tip portion, for example, the first lifting thread F, may be 1/10 or more greater than the elastic modulus of the lifting thread B of the rear end, for example, an nth lifting thread B. Therefore, by using the multi-stage lifting thread M by connecting a plurality of lifting threads in series during the lifting procedure, the lifting thread F at the front end of the lifting thread M may better withstand the frictional force against skin tissue than when using a single type of lifting thread (lifting thread according to the first and/or second example embodiment described above). At this time, the connecting portion connecting the adjacent lifting threads may be connected through an anchor at the end of the lifting thread, or may also be connected by various methods such as fusion or bonding.

[Advantageous Effects]

As described above, the present disclosure has the following effects.

First, by twisting a medical thread and then winding it around the outer peripheral surface (circumference) of a needle to manufacture a lifting thread in a coil spring shape, when the lifting thread is tensioned or compressed during a skin lifting procedure, a first elastic force according to the torsional stress generated by twisting the medical thread and a second elastic force that is the combined tensile or compressive stress generated when the lifting thread is tensioned or compressed during the skin lifting procedure are applied so that, even after a long time has passed after the skin lifting procedure, a significant amount of elasticity remains so that sagging of the lifted skin does not occur at all and the elasticity of the skin is maintained as it is, thereby maximizing the lifting effect.

Second, normally, the lifting thread goes through a process of increasing elasticity through heat treatment. However, when the heat treatment temperature of the lifting thread is too high as in the past (exceeding 75% of the melting temperature, for example, exceeding 85°C in the case of polydioxanone polymer), the elasticity of the lifting thread is improved, but during the skin lifting procedure, the lifting thread embedded inside the skin layer takes too short a time for hydrolysis, and thus it is easily hydrolyzed and the duration of the procedure effect is shortened. However, the lifting thread of the present disclosure is manufactured in a state of high elasticity by subjecting the medical thread to first twisting in one direction and then winding (coiling) it in the same direction or opposite to the twisting direction of the medical thread to have tensile or compressive coil spring characteristics. Therefore, there is no need to treat the heat treatment temperature of the lifting thread to a high temperature (exceeding 75% of the melting temperature, for example, exceeding 85°C in the case of polydioxanone polymer) more than necessary during the heat treatment of the lifting thread. Thus, during the skin lifting procedure as described above, the time for hydrolysis of the lifting thread embedded in the skin layer is shortened. Therefore, the disadvantage of being easily hydrolyzed and thus shortening the duration of the treatment effect can be fundamentally resolved.

Third, when the lifting thread of the composite coil spring structure is applied to the nasal cavity (nose) of the human body, the compression spring part of the lifting thread pushes up the tip of the nose to maintain a straight nose, while the tension spring part is tightly attached to each other like a pillar (base) to firmly support the entire lifting thread, enabling a stable procedure.

Fourth, the skin lifting procedure is safe because there is no risk of the lifting threads becoming permanently deformed and losing elasticity due to carelessness.

Fifth, when the lifting thread is embedded inside the skin layer, the lifting thread is supported by the non-slip combination portion so that it is smoothly inserted inside the skin layer without being pushed back, and when the needle is pulled out from the skin layer while the lifting thread is embedded inside the skin layer, the lifting thread stretches and generates elastic restoring force, but the non-slip combination portion prevents the lifting thread from moving arbitrarily in the needle, thereby effectively preventing the tensile length of the lifting thread from changing.

BRIEF DESCRIPTION OF THE DRAWINGS

FIG. 1 is a conceptual diagram for describing a lifting thread and lifting method disclosed in the related art in the document in the related art.

FIG. 2 is a combined perspective view showing a lifting thread insertion device according to a first example embodiment of the present disclosure.

FIG. 3 is a perspective view showing a lifting thread (tensile coil spring structure) of FIG. 2.

FIG. 4 is a view showing anchors provided on the tip and rear end portion of a lifting thread in a lifting thread insertion device according to the first example embodiment of the present disclosure.

FIG. 5 is a view showing a state in which, in the lifting thread insertion device according to the first example embodiment of the present disclosure, when the anchors are pulled in opposite directions during the skin lifting procedure, the anchors are caught inside the skin layer by hook jaws of the anchors, thereby continuously maintaining the tension of the lifting thread.

FIGS. 6 to 8 are views showing modified examples of the anchors, the anchors of FIG. 6 is of a spiral coupling type, and the anchors of FIG. 7 is of an ultrasonic fusion type, and the anchor of FIG. 8 is of a forced fit type.

FIG. 9 is a view showing a temporary knot formed on the hook jaw of an anchor provided at the rear end portion of the lifting thread in the lifting thread insertion device according to the first example embodiment of the present disclosure.

FIG. 10 is a side view showing a sliding holder and a withdrawal portion in the lifting thread insertion device according to the first example embodiment of the present disclosure.

FIGS. 11 and 12 are side views showing a non-slip combination portion of the lifting thread insertion device according to the first example embodiment of the present disclosure.

FIG. 13 is a combined perspective view showing a lifting thread insertion device according to a second example embodiment of the present disclosure, and FIG. 14 is a perspective view showing a lifting thread (compressive coil spring structure) of FIG. 13.

FIG. 15 is a combined perspective view showing a lifting thread insertion device according to a third example embodiment of the present disclosure.

FIG. 16 is a perspective view showing a lifting thread (composite coil spring structure) of FIG. 15.

FIG. 17 is a flowchart for describing a method for manufacturing a lifting thread (tensile coil spring structure) according to the first example embodiment of the present disclosure.

FIG. 18 is a flowchart for describing a method for manufacturing a lifting thread (compressive coil spring structure) according to the second example embodiment of the present disclosure.

FIG. 19 is a flowchart for describing a method for manufacturing a lifting thread (composite coil spring structure) according to the third example embodiment of the present disclosure.

FIG. 20 is a view showing a function of a lifting thread (composite coil spring structure) when the lifting thread is embedded in the nasal cavity (nose) according to the third example embodiment of the present disclosure.

FIG. 21 is a view showing a lifting thread (tensile coil spring structure) according to the first example embodiment of the present disclosure.

FIG. 22A is a view showing a lifting thread (compressive coil spring structure) according to the second example embodiment of the present disclosure.

FIG. 22B is a photograph showing a multi-stage lifting thread according to the first and/or second example embodiment of the present disclosure.

FIG. 23 is a view showing a lifting thread (composite coil spring structure) according to the third example embodiment of the present disclosure.

FIG. 24 is a surface photograph of a lifting thread of a tensile coil spring structure according to Example 1 of the present disclosure.

FIG. 25 is a surface photograph of a lifting thread of a tensile coil spring structure according to Example 2 of the present disclosure.

FIG. 26 is a surface photograph of a lifting thread of a tensile coil spring structure according to Example 3 of the present disclosure.

FIG. 27 is a surface photograph of a lifting thread of a tensile coil spring structure according to Example 4 of the present disclosure.

FIG. 28 is a surface photograph of a lifting thread of a tensile coil spring structure according to Example 5 of the present disclosure.

FIG. 29 is a surface photograph of a lifting thread of a tensile coil spring structure according to Example 6 of the present disclosure.

FIG. 30 is a surface photograph of a lifting thread of a tensile coil spring structure according to Example 7 of the present disclosure.

FIG. 31 is a surface photograph of a lifting thread of a tensile coil spring structure according to Example 8 of the present disclosure.

FIG. 32 is a surface photograph of a lifting thread of a tensile coil spring structure according to Comparative Example 1 of the present disclosure.

FIG. 33 is a graph showing the tensile stresses according to the tensile strains of the lifting threads according to Examples 1, 3, and 5 of the present disclosure.

FIG. 34 is a graph showing the Young's modulus according to the reverse twist angles of the lifting threads according to Examples 1 to 8 and Comparative Example 1 of the present disclosure.

## DETAILED DESCRIPTION

Hereinafter, a lifting thread, a method for manufacturing the lifting thread, and a lifting thread insertion device according to a preferred example embodiment of the present disclosure will be described in detail with reference to the attached drawings.

The present disclosure may have various modifications and may have various embodiments, and specific embodiments are illustrated in the drawings and described in detail.

Before the description of the present disclosure, the specific structural or functional descriptions below are merely examples for the purpose of describing example embodiments according to the concept of the present disclosure, and example embodiments according to the concept of the present disclosure may be implemented in various forms and should not be construed as being limited to the embodiments described in the present specification.

Further, in the description of this example embodiment, the descriptions of "first," "second," and "third" are merely arbitrary for the convenience of description, and of course, the order can be set in reverse order.

In the description of the present disclosure, the first example embodiment relates to a lifting thread of a tensile coil spring structure, the second example embodiment relates to a lifting thread of a compressive coil spring structure, and the third example embodiment relates to a lifting thread of a tensile and compressive coil spring composite structure.

Briefly look at the principle of the tensile coil spring structure of the present disclosure. Normally, when a tensile spring is pulled, the internal structure of the spring element wire receives the torsional stress and elastically deforms in one direction, and when the pulling force is removed, the twisting of the element wire is released and the increased length returns to its original state. That is, when a tension spring wound clockwise is pulled, the internal structure of the element wire undergoes a clockwise twisting deformation, and when the stretched spring is released, the element wire is untwisted counterclockwise, causing the stretched length of the spring to decrease again. By the same principle, when a tension spring wound counterclockwise is pulled, the internal structure of the element wire undergoes a counterclockwise twisting deformation, and when the stretched spring is released, the element wire is untwisted clockwise, causing the stretched length of the spring to decrease again.

However, the first example embodiment of the present disclosure is that the medical thread corresponding to the element wire of the tension spring is twisted in the same direction as the winding direction of the spring before being wound into a spring shape so that the internal structure of the medical thread is twisted in the same direction as when the length of the tension spring is increased in advance, and the medical thread is wound around the needle circumference from the tip toward the rear end portion to form a tension spring.

In the description of this example embodiment, in the process of winding and forming the medical thread around the circumference of the needle, the twist direction of the medical thread and the coiling direction of the spring may be determined differently depending on whether the direction in the process is described or the direction to be observed in the final product is described, such as when winding the medical thread while the needle is fixed, when winding the needle while the medical thread is fixed, when winding the medical thread while the tip of the needle is fixed, when winding the medical thread while the rear end portion of the needle is fixed, when the first end of the medical thread is fixed and the second end is rotated, when the second end of the medical thread is fixed and the first end is rotated, and when both ends of the medical thread are rotated in different directions.

Therefore, when the lifting thread according to this first example embodiment is pulled, the elastic force according to the length extension of the spring and the torsional elastic force due to the pre-twisting of the medical thread are combined to generate a stronger elastic restoring force, that is, the first elastic force described below, compared to a general spring-type lifting thread in which the medical thread is not pre-twisted. At this time, the elastic restoring force of the lifting thread according to the first example embodiment can be adjusted by increasing or decreasing the twist angle or the number of twisting turns of the medical thread.

At this time, a twist angle $\theta$ of the lifting thread may be defined as an angle formed by a twist line C that deforms as a parallel line that appears in a longitudinal direction on the surface of the untwisted medical thread is twisted around a longitudinal central axis L (imaginary line) of the medical thread and the central axis L. The twist angle $\theta$ may be experimentally determined by process variables such as the twist rotation angle and the number of twist rotations during the process of twisting the medical thread. When the twist angle $\theta$ is 0 degrees, it means that the medical thread is not twisted. In one specific example, the twist angle $\theta$ according to the first example embodiment may be greater than 0 degrees and less than 90 degrees.

The elastic modulus of the lifting thread may be adjusted by adjusting the twist angle $\theta$, and specifically, as the twist angle $\theta$ increases, the elastic modulus of the lifting thread may increase.

However, this twist angle $\theta$ may not change depending on the direction in which the medical thread is twisted, and the amount of twisting rotation and/or the number of twisting rotations, the twisting rotation angle, or the twisted medical thread may change in the process of being wound into a spring. For example, when the medical thread is twisted counterclockwise, the direction of the twist line C appearing on the surface of the medical thread may be a direction from the lower left to the upper right Z-twist, and when the medical thread is twisted clockwise, the direction of the twist line C may be a direction from the upper left to the lower right (S-twist).

In addition, the above-described number of twists refers to the number of times that one end of a unit-length medical thread is fixed and the other end is rotated by the twist angle, and may represent the degree of twist of the medical thread, that is, the degree of twist and the amount of twist. At this time, the number of twists may include a case where the angle of rotation based on the longitudinal central axis of the medical thread before twisting, that is, the twist angle is greater than 0 degrees and less than or equal to 360 degrees. For example, as the number of twists of the medical thread according to the first example embodiment increases, the medical thread is twisted more and the twist angle $\theta$ increases.

Therefore, as the number of twisting turns of the medical thread according to the first example embodiment increases, the twist angle $\theta$ increases, and the torsional stress of the medical thread, that is, the first torsional stress or first elastic force described later, increases.

In addition, depending on variables such as the twist angle $\theta$, the number of twist rotations, the twist rotation angle, the thickness of the medical thread, and the outer diameter of the needle to be coiled, there is also an effect of controlling the increase or decrease of the first elastic force due to the torsional stress generated by twisting the medical thread and/or the second elastic force, which is the first elastic force plus the tensile stress of the spring.

When a typical polymer spring undergoes a heat treatment process, its elasticity is improved and the medical thread is given the property of trying to maintain its coiled state. At this time, although the elasticity is improved as the heat treatment temperature increases, the time until hydrolysis after implantation in the human body is shortened. Thus, there is a disadvantage in that it is easily hydrolyzed and the treatment effect is shortened.

However, since the lifting thread according to the first example embodiment is manufactured with increased elasticity by pre-twisting the medical thread, there is no need to increase the heat treatment temperature, and it can have sufficient elasticity even when heat treated at a low temperature of 45% or less of the melting temperature (low temperature of 50°C or less in the case of polydioxanone). Therefore, the time until hydrolysis after being embedded in the human body is long, and thus the treatment effect lasts a long time, which is an advantage. At this time, the lifting thread according to the first example embodiment is a medical thread that maintains its coiled or twisted state after going through a heat treatment process, but at this time, the internal structure of the medical thread is already twisted. Therefore, it is further twisted when the spring length is extended, and thus the resistance to twisting (elasticity) is that much greater. This is the principle used.

Also, briefly looking at the principle of the compressive coil spring structure of the present disclosure, when a compression spring is compressed, the internal structure of the spring element wire receives the torsional stress and elastically deformsin one direction, and when the compressive force is removed, the twist of the element wire is released and the compressed length returns to its original state. That is, when a compression spring wound clockwise is compressed, the internal structure of the element wire is twisted counterclockwise, and when the compressed spring is released, the element wire is untwisted clockwise, and the shortened length of the spring is extended again. On the other hand, when a compression spring wound counterclockwise is compressed, the internal structure of the element wire is twisted clockwise, and when the compressed spring is released, the element wire is untwisted counterclockwise, and the shortened length of the spring extends again.

The lifting thread 20 according to the second example embodiment of the present disclosure is a medical thread corresponding to the wire of the compression spring, which is twisted in one direction (unidirectional) before being wound into a spring shape, thereby forming a preset twist so that the internal structure of the medical thread is twisted in the opposite direction to when the length of the compression spring is compressed in advance, thereby strengthening the characteristics of the compression spring. At this time, the twist direction of the medical thread according to the second example embodiment may be a direction opposite to that of the first example embodiment described above. That is, the medical thread is pre-twisted in the same direction as the internal tissue twist that occurs when the length of the compression spring is compressed and then wound around the needle circumference. Since the compression spring and the internal tissue twist are performed in a direction opposite to that of the tension spring, the twist direction of the medical thread according to the second example embodiment may be the direction opposite to that of the first example embodiment described above. Accordingly, the twist angle $\theta$ of the lifting thread 20 according to the second example embodiment may appear in the position opposite to that of the first example embodiment described above.

Meanwhile, the second example embodiment of the present disclosure may be formed by winding the medical thread corresponding to the element wire of the compression spring in the shape of a spring in a direction opposite to the winding direction of the spring before winding it, thereby creating resistance to shear deformation (torsion) that occurs when compressing the coil spring, and then winding the medical thread from the tip toward the rear end portion at the circumference of the needle to form a compression spring. At this time, by increasing the axial compressive deformation resistance of the spring, there is an effect of increasing the elasticity of the compression spring, specifically, the elastic modulus.

In the description of this example embodiment, in the process of winding and forming the medical thread around the circumference of the needle, the twist direction of the medical thread and the coiling direction of the spring may be determined differently depending on whether the direction in the process is described or the direction to be observed in the final product is described, such as when winding the medical thread while the needle is fixed, when winding the needle while the medical thread is fixed, when winding the medical thread while the tip of the needle is fixed, when winding the medical thread while the rear end portion of the needle is fixed, when the first end of the medical thread is fixed and the second end is rotated, when the second end of the medical thread is fixed and the first end is rotated, and when both ends of the medical thread are rotated in different directions.

Therefore, when the lifting thread according to this second example embodiment is compressed, the elastic force due to the compression of the spring's length and the torsional elastic force due to the pre-twisting of the medical thread are combined to generate a stronger elastic restoring force, that is, a second elastic force described below. In this case, the lifting thread according to the second example embodiment may also have its elastic restoring force adjusted by increasing or decreasing the twist angle $\theta$ or the number of twisting turns of the medical thread.

At this time, the twist angle $\theta$ according to this second example embodiment may be greater than 0 degrees and less than 90 degrees.

In addition, as the number of twisting turns of the medical thread according to the second example embodiment increases, the medical thread twists more, the twist angle $\theta$ increases, and the torsional stress of the medical thread, that is, the first torsional stress described below, increases.

In addition, there is an effect that can control the increase or decrease of the first elastic force due to the torsional stress generated by twisting the medical thread according to variables such as the twist angle $\theta$, the number of twist rotations, and the twist rotation angle.

Therefore, since the lifting thread 20 according to the second example embodiment is manufactured with increased elasticity by pre-twisting the medical thread, there is no need to increase the heat treatment temperature, and it can have sufficient elasticity even when heat treated at a low temperature of 45% or less of the melting temperature (low temperature of 50°C or less in the case of polydioxanone). Therefore, the time until hydrolysis after being embedded in the human body is long, and thus the treatment effect lasts a long time, which is an advantage.

In addition, a lifting thread 30 having a tensile and compressive coil spring composite structure according to the third example embodiment of the present disclosure and its principle will be briefly examined. The tensile and compressive coil spring composite structure has one portion having a tensile coil spring structure and at least one other portion having a compressive coil spring structure. That is, the lifting thread 30 according to the third example embodiment of the present disclosure may be described as two or more coil springs that operate independently, the tensile coil spring according to the first example embodiment and the compressive coil spring according to the second example embodiment, connected through the same medical thread.

The lifting thread 30 according to the third example embodiment of the present disclosure may include a composite structure in which a first coil spring structure in which a first twisted thread in which a portion of a single or a plurality of medical threads is twisted is wound (coiled) and a second coil spring structure in which a second twisted thread in which another portion of the medical threads is twisted in the opposite direction is wound (coiled) are connected so that they can operate independently. In the composite structure lifting thread 30, the first twist angle, which is an angle at which the first twisted thread is twisted, and the second twist angle, which is an angle at which the second twisted thread is twisted, are each independently adjusted to adjust the elastic modulus of the composite structure lifting thread 30.

Specifically, the lifting thread 30 according to the third example embodiment of the present disclosure may be formed by subjecting one side of a medical thread to first twisting in one direction (first twisted thread), then subjecting the medical thread to first winding in a tensile coil spring shape on the outer peripheral surface of the needle 1 in the same direction as the twisting direction of the medical thread (first coil spring structure), and then the remaining portion of the medical thread to second twisting in the opposite direction so that the torsional stress opposite to the first torsional stress is generated (second twisted thread), and then winding the medical thread in a compressed coil spring shape on the outer peripheral surface of the needle 1 in the same direction as the first twisting direction (second coil spring structure). Accordingly, the structure of the lifting thread 30 according to the third example embodiment has a portion having a tensile coil spring structure and a remaining portion having a compressive coil spring structure. For example, the first coil spring structure may be a tensile coil spring structure, and the second coil spring structure may be a compressive coil spring structure. In addition, the first twisted thread and the second twisted thread may be the same twisted thread, or may be a thread in which different twisted threads are attached and connected.

In the description of this example embodiment, the twist direction of the medical thread and the coiling direction of the spring may be determined differently depending on the various changes in the process described above during the process of winding and forming the medical thread around the circumference of the needle, the direction during the process, or the direction observed in the final product.

In addition, there is an effect that can control the increase or decrease of the first elastic force due to the torsional stress generated by twisting the medical thread according to variables such as the twist angle $\theta$, the number of twist rotations, and the twist rotation angle.

The lifting thread 30 according to the third example embodiment of the present disclosure may have one portion that acts as a tensile spring that pulls the skin tissue, and the remaining part that acts as a compression spring that pushes the skin tissue so that the tensile or compressive elasticity of the skin tissue may be controlled according to the condition of the skin tissue by using the principle of pushing some parts and pulling some parts.

At this time, the lifting thread 30 according to this third example embodiment may have independent twist angles $\theta$ since the first and second twisting steps are performed on the medical thread in the manufacturing method thereof. For example, the lifting thread 30 according to the third example embodiment may include a first twist angle $\theta 1$ derived from the first twisting step and appearing in the first twisted thread, and a second twist angle $\theta 2$ derived from the second twisting step and appearing in the second twisted thread. The first twist angle $\theta 1$ and the second twist angle $\theta 2$ are derived from independent twist steps and may have the same value or different values. Therefore, the lifting thread 30 according to the third example embodiment may have at least one twist angle $\theta$, and the twist angle $\theta$ may be the first twist angle $\theta 1$ or the second twist angle $\theta 2$. Since the twist angle $\theta$ may be independently controlled, a plurality of elastic moduli may be simultaneously provided in the same lifting thread 30, and the tensile or compressive elasticity of the skin tissue may be customized and controlled depending on the state of the skin tissue.

Meanwhile, the lifting thread 10 or 20 according to the first, second or third example embodiment may also form a multi-stage lifting thread by connecting a plurality of lifting threads in series. The multi-stage lifting thread M may be a plurality of independent lifting threads that are the same or different from each other connected in series, and specifically, the multi-stage lifting thread M may be a number of n independent lifting threads that are the same or different from each other connected in series through n-1 connecting portions (not shown) (wherein n is any positive integer). The multi-stage lifting thread M may have a higher elastic modulus of the lifting thread F located at the front with respect to the entry direction of the lifting thread during the lifting procedure than the lifting thread B located at the rear. For example, the elastic modulus of the lifting thread F at the front end, for example, the first lifting thread F, may be 1/10 or more higher than the elastic modulus of the lifting thread B at the rear end, for example, an nth lifting thread B. Therefore, by using the multi-stage lifting thread M by connecting a plurality of lifting threads in series during a lifting procedure, the lifting thread F at the front end of the lifting thread M may better withstand the frictional force against skin tissue than when using a single type of lifting thread (lifting thread according to the first and/or second example embodiment described above). At this time, the connecting portion connecting the adjacent lifting threads may be connected through an anchor at the end of the lifting thread, or may also be connected by various methods such as fusion or bonding.

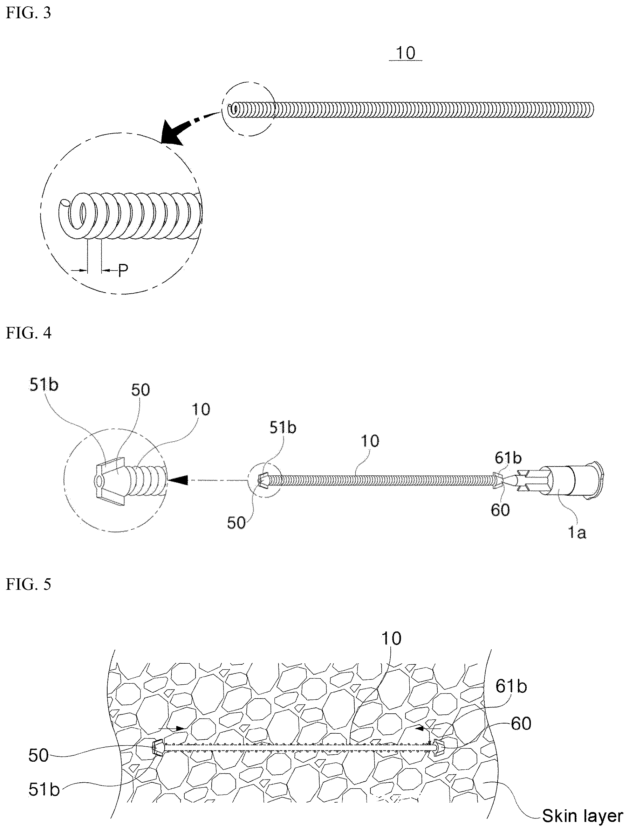

First, FIG. 2 is a combined perspective view showing the lifting thread insertion device according to the first example embodiment of the present disclosure, and FIG. 3 is a perspective view showing the lifting thread (tensile coil spring structure) of FIG. 2.

Referring to FIGS. 2 and 3, a lifting thread insertion device 100 according to the first example embodiment of the present disclosure may include a needle 1, and a lifting thread 10 formed by winding (coiling) a medical thread from the tip of the outer peripheral surface of the needle 1 toward the rear end portion thereof in a tensile coil spring structure in the same direction as the twisting direction of the medical thread while twisting the medical thread in one direction so that the torsional stress is generated inside the medical thread (yarn).

The lifting thread insertion device 100 according to the first example embodiment of the present disclosure may be configured so that, during the skin lifting procedure, a first elastic force according to the torsional stress of the medical thread and a second elastic force, which is a combination of the tensile stress generated when the lifting thread 10 is tensioned, are applied. Here, the first elastic force of the lifting thread 10 may be configured to be adjusted by increasing or decreasing the twist angle of the medical thread.

In the lifting thread insertion device 100 according to the first example embodiment of the present disclosure, the needle 1 may be a bar shape with a filled interior, or when the inside of the needle body of a pipe shape is filled with a bar of a predetermined length, the strength of the needle 1 itself is reinforced so that the needle 1 is not cut during the lifting procedure, and thus there is an advantage in that the procedure can be performed safely.

The needle 1 may have a handle 1a formed on the rear end portion that may be held by an operator. The needle 1 may be usually made of iron core or the like, while the handle 1a is made of a synthetic resin or the like, but is not necessarily limited thereto. The shape and structure of the needle 1 are not limited to this example embodiment and may be changed into various shapes depending on the operating conditions or the like.

After the skin lifting procedure, only the lifting thread 10 in a coil spring shape remains elastically (embedded) in the skin layer while maintaining a predetermined pitch P. The reason why only the lifting thread 10 remains elastically in the skin layer while maintaining the predetermined pitch P is because of the skin tissue having a collagen fiber structure. That is, the collagen fibers of the skin layer are integrated so that they move together with the lifting thread 10 while wrapping inwardly along the outer peripheral surface of the lifting thread 10, and the skin is elastically lifted as it contracts due to the elastic restoring force of the lifting thread 10.

Meanwhile, FIG. 4 is a view showing anchors provided on the tip and rear end portion of a lifting thread in the lifting thread insertion device according to the first example embodiment of the present disclosure, and FIG. 5 is a view showing a state in which, in the lifting thread insertion device according to the first example embodiment of the present disclosure, when the anchors are pulled in opposite directions during the skin lifting procedure, the anchors are caught inside the skin layer by hook jaws of the anchors, thereby continuously maintaining the tension of the lifting thread.

As shown in FIGS. 4 and 5, the lifting thread insertion device 100 according to the first example embodiment of the present disclosure may further include anchors 50 and 60 as accessories provided to each of the tip and rear end portion of the lifting thread 10. For example, the tip of the lifting thread 10, which is first injected into the skin during a lifting procedure, may include the anchor 50, and the rear end portion, which is later injected, may include the anchor 60.

The shapes of the anchors are not limited to this example embodiment and may be changed in various ways, and one of these anchors may be selected and used depending on the treatment conditions.

When the anchors 50 and 60 are pulled in a direction facing each other, the anchors 50 and 60 are provided with hook jaws 51b and 61b so as to be caught inside the skin layer and continuously maintain the tension state of the lifting thread 10.

When the anchors 50 and 60 are pulled in a direction facing each other inside the skin layer, the hook jaws 51b and 61b are caught inside the skin layer to continuously maintain the tension state of the lifting thread 10.

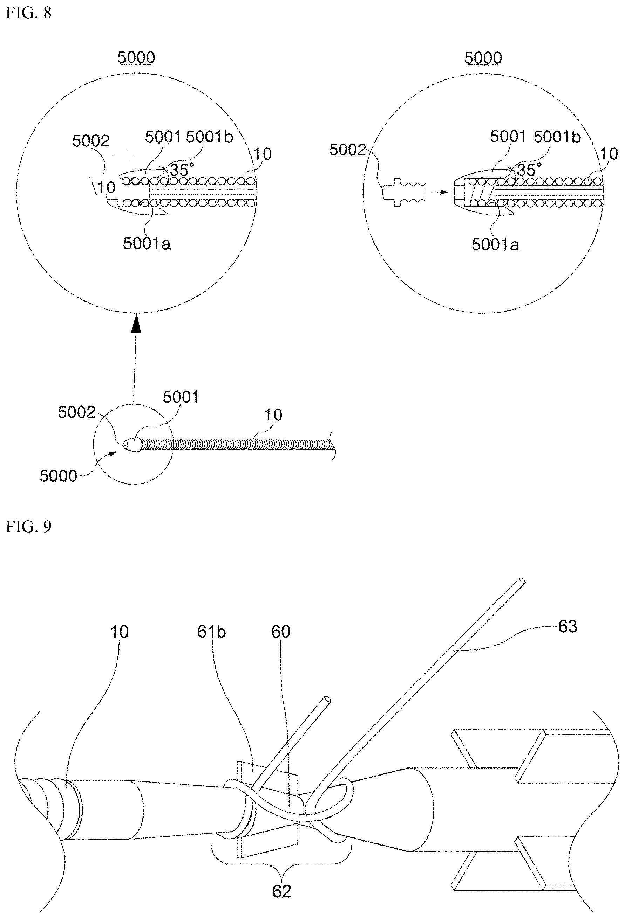

The shapes of the anchors may be formed in various ways, such as a cone (not shown), a cone with a neck (not shown), a cone with straight wings (see FIG. 4), a cone with straight arrowhead wings (not shown), a cone with cross wings (not shown), a cone or a cone with a neck with straight arrowhead wings (see FIG. 9).

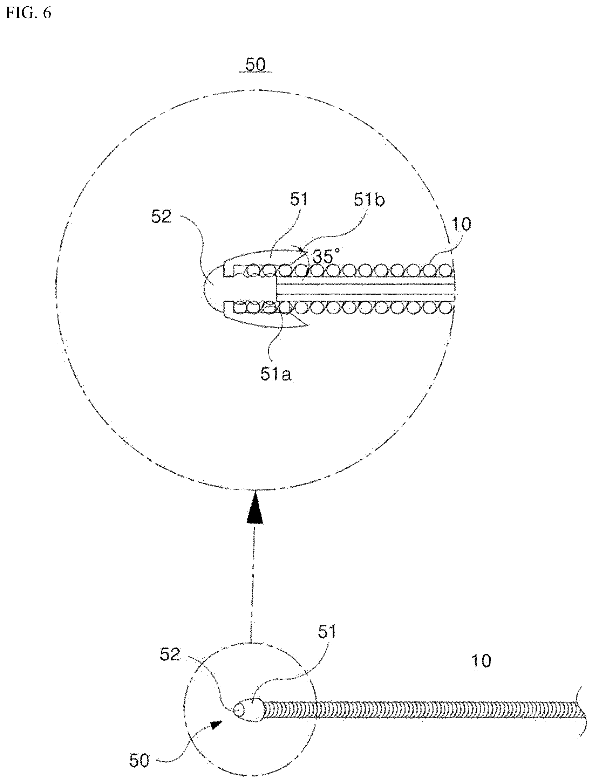

Also, FIGS. 6 to 8 are views showing modified examples of anchors.

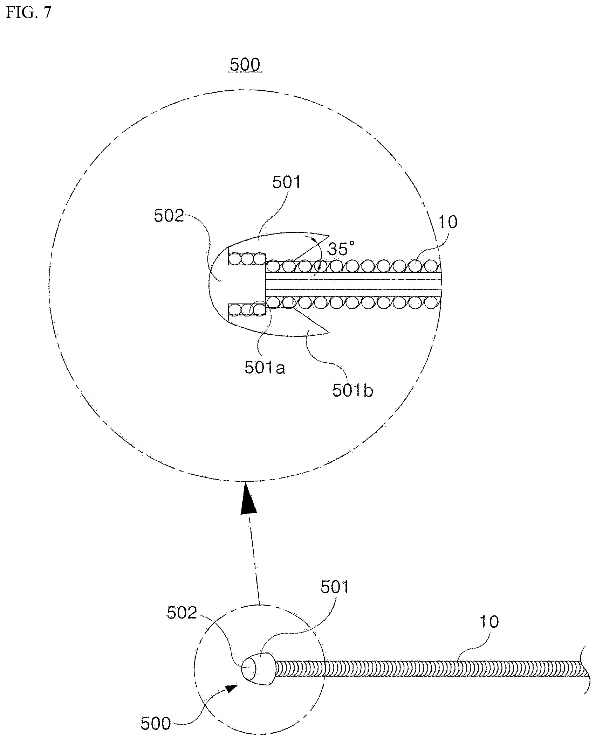

The anchor 50 may be of a spiral coupling type (shown in FIG. 6), an anchor 500 may be of an ultrasonic fusion type (shown in FIG. 7), and an anchor 5000 may be of a forced fit type (shown in FIG. 8).

The anchors 50, 500, and 5000 shown in FIGS. 6 to 8 may include fastening members 52, 502, and 5002 that are coupled to the inner circumference of the tip of the lifting thread 10 and anchor bodies 51, 501, and 5001 that are covered on the outer circumference of the tip of the lifting thread 10, respectively, in which the lifting thread 10 may be fixed between the fastening members 52, 502, and 5002 and the anchor bodies 51, 501, and 5001 using a spiral connection type, an ultrasonic fusion type, or a forced fit type.

The anchors 50, 500, 5000, and 60 shwon in FIGS. 6 to 9 are coupled to the tip (or rear end portion) of the lifting thread 10 and is provided with hook jaws 51b, 501b, 5001b, and 61b that protrude radially outwardly more than the outer diameter of the lifting thread 10, respectively. For example, the angle of each of the hook jaws 51b and 61b may be set to 35°, but is not necessarily limited thereto.

When the anchor 50, 500, 5000, or 60 described above embeds the lifting thread 10 inside the skin layer, the hook jaw 51b, 501b, 5001b, or 61b of the anchor 50, 500, 5000, or 60 is caught inside the skin layer and serves to fix the lifting thread 10 in a tensioned state.

To explain further, the anchor body 51 of FIG. 6 may have a spiral groove 51a formed in the center and is fixed to the outer surface of the lifting thread 10 by a screw connection, and a hook jaw 51b that catches on the skin layer is formed at the circumference of the anchor body 51 to serve to fix the lifting thread 10 embedded inside the skin layer in a tensioned state.

An anchor body 501 in FIG. 7 may have a spiral groove 501a formed in the center and is fixed to the outer surface of the lifting thread 10 by ultrasonic fusion type, and a hook jaw 501b that catches on the skin layer is formed at the circumference of the anchor body 501 to serve to fix the lifting thread 10 embedded inside the skin layer in a tensioned state.

Further, an anchor body 5001 of FIG. 8 may have a spiral groove 5001a formed in the center, and a fastening member 5002 is fixed in the spiral groove 5001a by a forced fit, and a hook jaw 5001b that catches on the skin layer is formed at the circumference of the anchor body 5001 to serve to fix the lifting thread 10 embedded in the skin layer in a tensioned state.

In addition, the anchor 50, 500, or 5000 may be formed integrally with the lifting thread 10. At this time, one of the anchor 50, 500, or 5000 may be provided at each of the tip and the latter half of the spring section, or a plurality of anchors may be formed at regular intervals to be firmly attached to the skin tissue.

In addition, FIG. 9 is a view showing a temporary knot 62 formed on the hook jaw 61b of the anchor 60 provided on a rear end portion of the lifting thread 10 in the lifting thread insertion device 100 according to the first example embodiment of the present disclosure.

Referring to FIG. 9, the anchor 60 may provided with the hook jaw 61b that catches the skin layer at its circumference to serve to fix the lifting thread 10 embedded in the skin layer in a tensioned state. Particularly, unlike the anchor 50 included in the tip of the lifting thread 10 that is first injected into the skin during a lifting procedure, the wing direction of the hook jaw 61b of the anchor 60 included in the rear end portion that is injected later may be formed in a direction opposite to a direction in which the lifting thread 10 enters the skin, and physical interference may occur during the process in which the hook jaw 61b is embedded into the skin layer. Therefore, in order to eliminate physical interference that occurs during the process of inserting the lifting thread insertion device 100 into the skin layer during a lifting procedure and to smoothly insert the lifting thread 10 into the skin layer, the temporary knot 62 may be additionally included to fill the gap (or groove) between the hook jaw 61b and the lifting thread 10. The temporary knot 62 may be formed by winding a thread 63 for the temporary knot around the portion where the gap is formed to fill the gap (or groove) between the protruding portion of the hook jaw 61b, for example, a wing or arrowhead shape, and the lifting thread 10 and to minimize the step between the hook jaw 61b and the lifting thread 10. In addition, the temporary knot 62 may be knotted in a way that it is easily untied when one end of the temporary knot 62 is pulled so that it is removed without remaining inside the skin layer after the lifting thread 10 is inserted into the skin layer. Various knotting methods may be applied, and for example, a knotting method in which the short end of the temporary knot 62 comes out of the temporary knot 62 and is untied when the long end of the temporary knot 62 is pulled may be applied, but is not necessarily limited thereto. At this time, the thread 63 for the temporary knot may use the medical thread described above, or any other type of medical thread that may be used for medical purposes may be used.

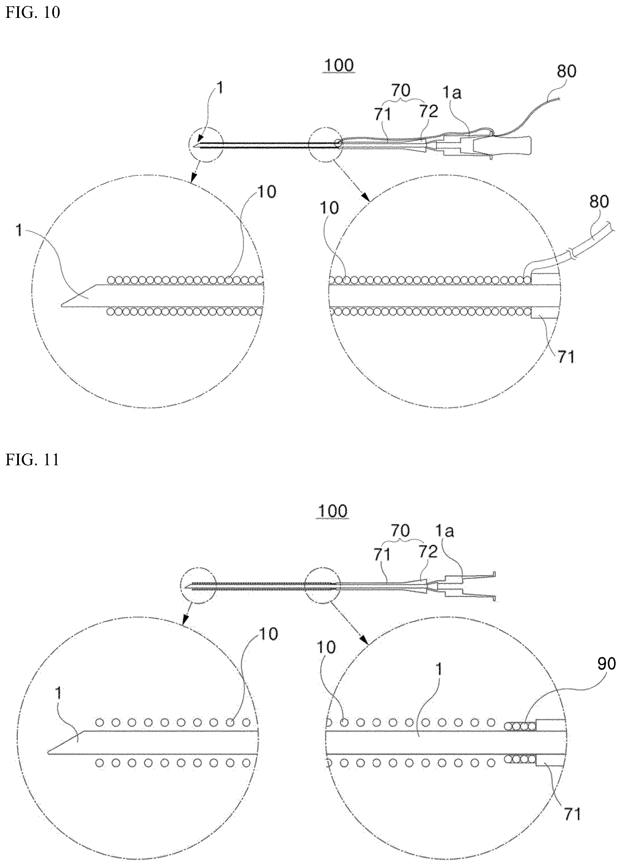

Meanwhile, FIG. 10 is a side view showing the sliding holder and the withdrawal portion in the lifting thread insertion device 100 according to the first example embodiment of the present disclosure.

As shown in FIG. 10, the lifting thread insertion device 100 according to the first example embodiment of the present disclosure may further include a pipe shape, which is slidably coupled to the outer peripheral surface of the needle 1 in a forward and backward manner, and a sliding holder 70 that supports the rear end portion of the lifting thread 10 so that, when the needle 1 is pulled out from the skin layer during the skin lifting procedure, the lifting thread 10 does not come out of the skin layer together with the needle 1 but remains embedded in the skin layer, and a withdrawal portion 80 that is connected to the rear end portion of the lifting thread 10 so that, when the lifting thread 10 is embedded in the skin layer, the lifting thread 10 may be pulled and tensioned by being exposed to the outside of the skin layer by a predetermined length.

The withdrawal portion 80 is a thread that is cut and removed after the lifting thread 10 is pulled out of the skin layer during a skin lifting procedure, and typically extends from the rear end portion of the lifting thread 10. It is a separate thread that is extended long in the rear so that the lifting thread 10 may be exposed to the outside of the skin layer even after being embedded inside the skin layer and can be easily pulled by the operator.

The lifting thread 10 and the withdrawal portion 80 are not necessarily configured separately, and in some cases, they may be formed as an integral part with the lifting thread 10 so as to extend rearward in a longitudinal direction of the needle from the rear end portion of the lifting thread 10.

In addition, although not specifically shown in the drawing, the withdrawal portion 80 needs to be fixed after tensioning the lifting thread 10. To this end, it is preferable to form a negative or positive protrusion on the surface of the withdrawal portion 80 to facilitate fixing of the withdrawal portion 80.

The lifting thread 10 may be manufactured to maintain the coil spring shape and have elasticity through a heat treatment process. During a skin lifting procedure, the lifting thread 10 may be pulled by the withdrawal portion 80 and remain elastically inside the skin layer (embedded) while maintaining the predetermined pitch P. At this time, the lifting thread 10 may be inserted into the skin layer while having accumulated elasticity, and the pitch P of the lifting thread 10, which has been elastically changed, is restored to its original state by the force (elastic restoring force) to lift more tissues with weak elasticity according to the elasticity of the skin layer, thereby obtaining a more natural lifting effect. Furthermore, since the surface of the lifting thread 10 does not have thorns or protrusions, it has the effect of significantly reducing a patient's pain.

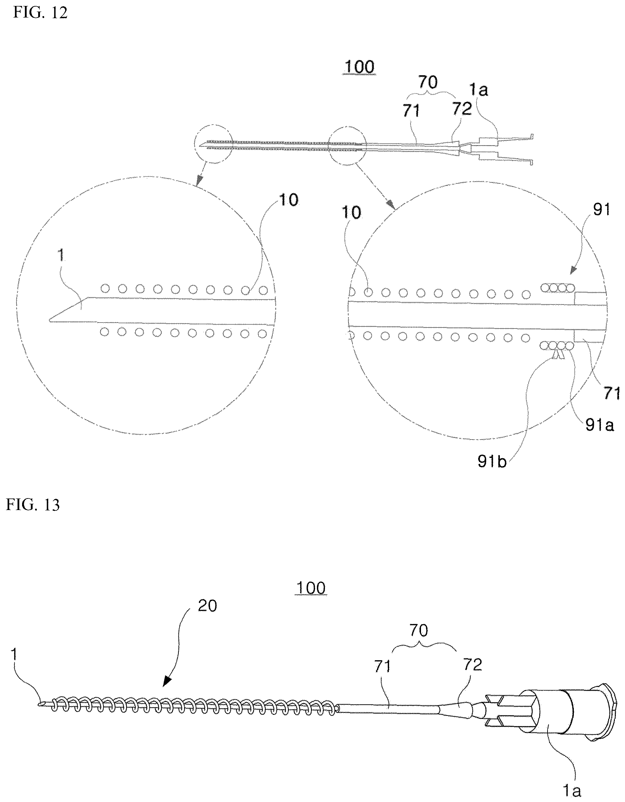

Meanwhile, FIGS. 11 and 12 are side views showing a non-slip combination portion of the lifting thread insertion device 100 according to the first example embodiment of the present disclosure.

As shown in FIGS. 11 and 12, the lifting thread insertion device 100 according to the first example embodiment of the present disclosure may be formed on the rear end portion of the lifting thread 10 and may be relatively tightly coupled to the outer peripheral surface of the needle 1 compared to other portions, and may serve to support the lifting thread 10 from being pushed backward when the needle 1 is inserted into the skin layer during a lifting procedure, and when the tip of the lifting thread 10 is fixed in position in the skin layer and the needle 1 is pulled backward, the rear end portion of the lifting thread 10 may be pulled out together with the needle 1 and may be stretched to have elastic restoring force. It may further include a non-slip combination portion 90 or 91.

At this time, the rear of the non-slip combination portion 90 or 91 may be configured to further include the sliding holder 70 formed in a pipe shape so as to be slidable back and forth on the outer peripheral surface of the needle 1, and capable of supporting the rear end portion of the lifting thread 10 when separating the lifting thread 10 from the needle 1 during a lifting procedure and separating the needle 1 from the skin layer.

The non-slip combination portion 90 or 91 may be tightly combined with the outer peripheral surface of the needle 1 so that it does not slip from the needle 1 and remains combined with the outer peripheral surface of the needle 1 when embedded in or pulled out of the skin layer.

As an example of the non-slip combination portion, the rear end portion of the lifting thread 10 may be configured to include a tight winding portion 90 that is wound relatively tightly (firmly) on the outer peripheral surface of the needle 1 compared to other portions.

Although not shown in the drawing, in forming the above-described close-winding portion 90, a rod-shaped mold (not shown) having a stepped portion with a small diameter at one end is prepared, and a medical thread is wound in a spiral shape around the circumference of the mold to form the lifting thread 10. As a result, the portion wound around the stepped portion has a relatively smaller spiral diameter than other portions, thereby forming the close-winding portion 90. When this lifting thread 10 is fitted around the circumference of the needle 1, the close-winding portion is tightly coupled to the needle circumference so as not to slip.

When the sliding holder 70 is provided at the rear of the lifting thread 10, it may be used very usefully when separating the lifting thread 10 from the needle 1, and it is also preferable because it my supplement the non-slip combination portion 90 when embedding the lifting thread 10 inside the skin layer.

In addition, as another example of the non-slip combination portion, the lifting thread 10 may be configured to include a knot portion 91a that more tightly couples the lifting thread 10 to the outer peripheral surface of the needle 1 through the intervention of a binding thread 91b passing between the lifting thread 10 and the needle 1.

The knot portion 91a may be tied by preparing a separate binding thread 91b and passing it between the lifting thread 10 and the needle 1 so that the lifting thread 10 is more tightly bonded to the outer peripheral surface of the needle 1 through the intervention of the binding thread 91b, thereby preventing slipping.

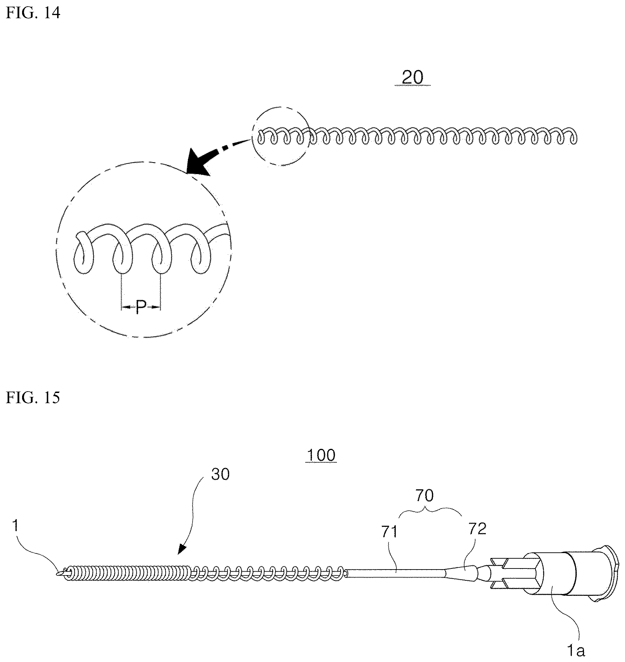

Meanwhile, FIG. 13 is a combined perspective view showing the lifting thread insertion device according to the second example embodiment of the present disclosure, and FIG. 14 is a perspective view showing the lifting thread (compressive coil spring structure) of FIG. 13.

Referring to FIGS. 13 and 14, a lifting thread insertion device (200) according to a second example embodiment of the present disclosure may include a needle 1, and a lifting thread 20 formed by winding a medical thread from the tip of the outer peripheral surface of the needle 1 toward the rear end portion thereof in a direction opposite to a twisting direction of the medical thread while twisting the medical thread in one direction so that the torsional stress is generated inside the medical thread (yarn) with a compressive coil spring structure.

The lifting thread insertion device 200 according to the second example embodiment of the present disclosure may be configured so that, when the lifting thread 20 is compressed during the skin lifting procedure, the lifting thread 20 is subjected to a first elastic force according to the torsional stress of the lifting thread 20 and a second elastic force that is a combination of the compressive stress generated when the lifting thread 20 is compressed.

Here, the first elastic force of the lifting thread 20 may be configured to be adjusted by changing the twist angle of the medical thread, the thickness of the medical thread or the like.

The lifting thread insertion device 200 according to the second example embodiment of the present disclosure may be formed in a pipe shape and may be connected to the outer peripheral surface (circumference) of the needle 1 so as to be able to slide forward and backward, and may further include the sliding holder 70 that, when the needle 1 is pushed into the skin layer, the lifting thread 20 is not pushed to the rear of the needle 1 but is embedded into the skin layer together with the needle 1 and is compressed inside the skin layer.

The sliding holder 70 may be configured to include a grip portion 71 for the operator to hold with his/her hand; and a sliding tube 72 extending from the grip portion 71 toward the tip of the needle and supporting the rear end portion of the lifting thread 10 during a lifting procedure.

The sliding tube 72 may be formed to extend toward the tip of the needle longer than the tensile length of the lifting thread 10, and may be configured to support the rear end portion of the lifting thread 10 so that the tip of the lifting thread 10 may be inserted to a desired location in the skin layer during the skin lifting procedure, while pushing the lifting thread 10 into the inside of the skin layer.

In addition, the lifting thread insertion device 200 according to the second example embodiment of the present disclosure may additionally include accessories included in the lifting thread insertion device 100 according to the first example embodiment described above, such as an anchor, a hook jaw, and the like.

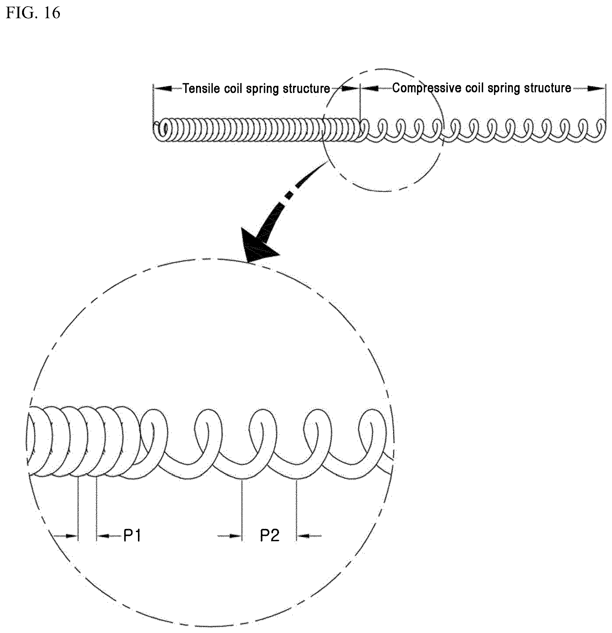

Meanwhile, FIG. 15 is a combined perspective view showing a lifting thread insertion device according to the third example embodiment of the present disclosure, and FIG. 16 is a perspective view showing the lifting thread (tensile and compressive coil spring structure) of FIG. 15.

Referring to FIGS. 15 and 16, a lifting thread insertion device 300 according to the third example embodiment of the present disclosure may include a needle 1, and a lifting thread 30 formed by subjecting the medical thread (yarn) to first twisting in one direction so that the torsional stress is generated inside the medical thread, and then subjecting the medical thread in a tensile coil spring shape to first winding on the outer peripheral surface of the needle 1 in the same direction as the twisting direction of the medical thread, and then the medical thread to second twisting in the opposite direction so that the torsional stress is generated inside the medical thread in a direction opposite to that of the first torsional stress, and then the medical thread in a compressed coil spring shape to second winding on the outer peripheral surface of the needle 1 in the same direction as the first twisting direction, in which the lifting thread 30 is configured to have a combination of tensile coil spring characteristics (structure) and compressive coil spring characteristics (structure).

Here, the elasticity according to the torsional stress of the lifting thread 30 may be configured to be adjusted according to changes in the twist angle of the medical thread, the thickness of the medical thread, the inner diameter of the lifting thread, the pitch, or a combination thereof.

In the skin lifting procedure, the tension coil spring portion of the lifting thread 30 inside the skin layer may have a dense pitch P1 to act as a pillar (base), and the compression coil spring portion has a non-dense pitch P2 to act as an elastic lifting portion of the skin layer.

In addition, the lifting thread insertion device 300 according to the third example embodiment of the present disclosure may additionally include accessories, such as an anchor, a hook jaw, and the like included in the lifting thread insertion device 100 according to the first example embodiment and/or the lifting thread insertion device 200 according to the second example embodiment.

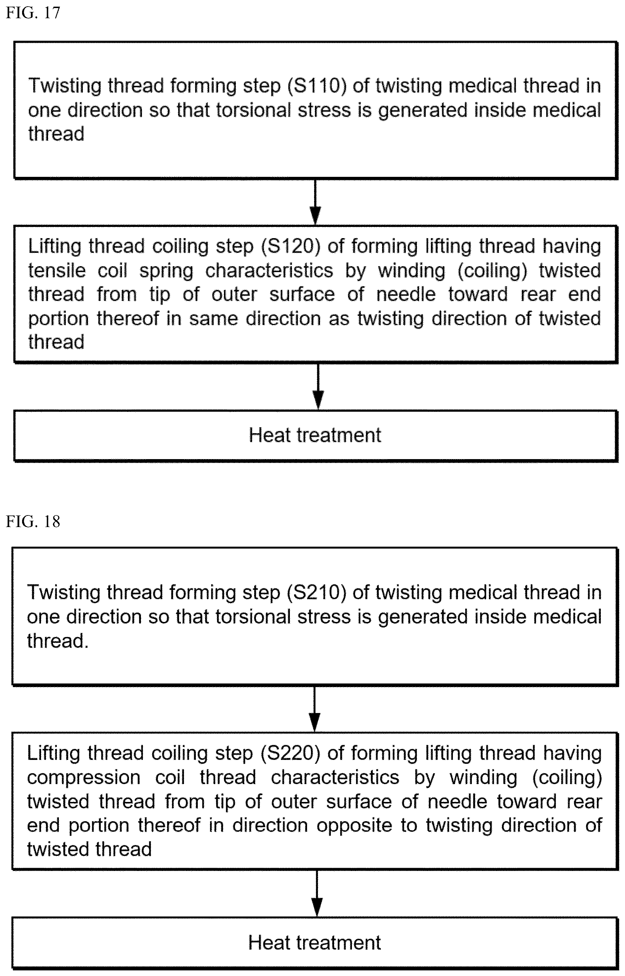

Meanwhile, FIG. 17 is a flowchart for describing a method for manufacturing the lifting thread 10 according to the first example embodiment of the present disclosure.

Referring further to FIG. 17, the method for manufacturing the lifting thread 10 according to the first example embodiment of the present disclosure may be a method for manufacturing the lifting thread 10 having tensile coil spring characteristics by twisting a medical thread (yarn) in one direction and then winding (coiling) it in the same direction as the twisting direction of the medical thread.

The method for manufacturing the lifting thread 10 according to the first example embodiment of the present disclosure may include a twisted thread forming step S110 of twisting the medical thread in one direction so that the torsional stress is generated inside the medical thread, and a lifting thread coiling step S 120 of winding (coiling) the twisted thread from the tip of the outer peripheral surface of the needle toward the rear end portion in the same direction as the twisting direction of the twisted thread to form the lifting thread 10 having tensile coil spring characteristics, in which, when tensioning the lifting thread 30 during the skin lifting procedure, the lifting thread 30 is subjected to a first elastic force according to the torsional stress of the lifting thread 30 and a second elastic force that is the result of the tensile stress of the lifting thread 30 being added thereto.

Also, FIG. 18 is a flowchart for describing a method for manufacturing the lifting thread 20 according to the second example embodiment of the present disclosure.

Referring further to FIG. 18, the method for manufacturing the lifting thread 20 according to the second example embodiment of the present disclosure may be a method for manufacturing the lifting thread 20 having compressive coil spring characteristics by twisting a medical thread in one direction and then winding (coiling) it in the opposite direction to the twisting direction of the medical thread.

The method for manufacturing the lifting thread 20 according to the second example embodiment of the present disclosure may include a twisted thread forming step S210 of twisting the medical thread in one direction so that the torsional stress is generated inside the medical thread, and a lifting thread coiling step S220 of forming the lifting thread 20 having compressive coil spring characteristics by winding (coiling) the twisted thread from the tip of the outer peripheral surface of the needle toward the rear end portion thereof in the direction opposite to the twisting direction of the twisted thread, in which, during the skin lifting procedure, a first elastic force according to the torsional stress generated by twisting the medical thread and a second elastic force in which the compressive stress of the lifting thread 20 is combined are applied.

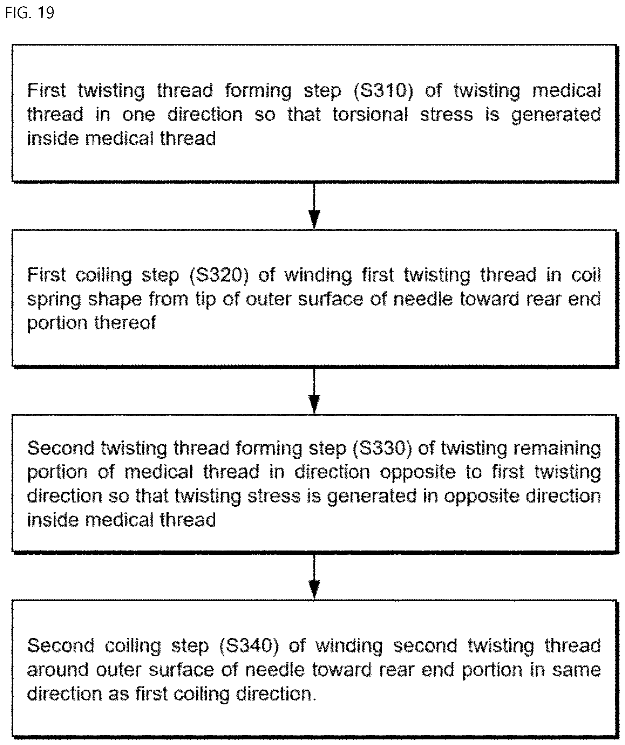

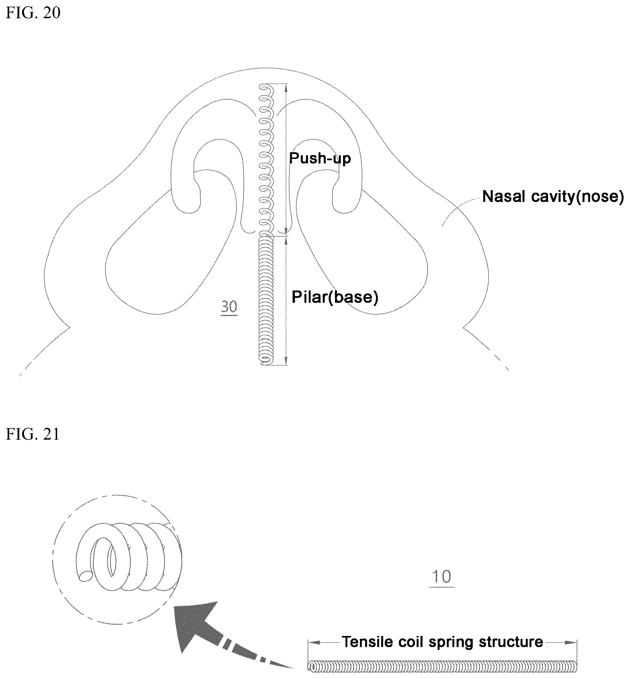

In addition, FIG. 19 is a flowchart for describing a method for manufacturing the lifting thread 30 according to the third example embodiment of the present disclosure, and FIG. 20 is a view showing ae function of the lifting thread 30 when the lifting thread 30 according to the third example embodiment of the present disclosure is embedded (inserted) in the nasal cavity (nose).

First, referring to FIG. 19, the method for manufacturing the lifting thread 30 according to the third example embodiment of the present disclosure may be a method for manufacturing the lifting thread 30 of a composite structure in which a portion of a medical thread is first wound in a tensile coil spring shape around the outer peripheral surface of the needle 1, and the remaining portion of the medical thread is second wound in a compressed coil spring shape around the outer peripheral surface of the needle 1.

A method for manufacturing the lifting thread (30) according to the third example embodiment of the present disclosure may be a method for manufacturing a composite structure lifting thread having tensile coil spring characteristics and compressive coil spring characteristics, and may include a first twisted thread forming step S310 of twisting the medical thread in one direction so that torsional stress is generated inside the medical thread, a first coiling step S320 of winding the first twisted thread into a coil spring shape from the tip of the outer peripheral surface of the needle 1 toward the rear end portion thereof, a second twisted thread forming step S330 of twisting the remaining portion of the medical thread in a direction opposite to the first twisting direction so that torsional stress is generated inside the medical thread in the opposite direction, and a second coiling step S340 of winding the second twisted thread in the same direction as the first coiling direction on the outer peripheral surface of the needle 1 toward the rear end portion thereof.

The lifting thread 30 according to the third example embodiment of the present disclosure may have one side that acts as a tension spring that pulls the skin tissue, and the other side that acts as a compression spring that pushes the tissue inside the skin layer so that it enables a composite treatment that pushes some parts and pulls some parts depending on the condition inside the skin layer.

In addition, the lifting thread 30 according to the third example embodiment of the present disclosure may have one side that acts as a pillar (base) of a building to firmly support the lifting thread 30 by having adjacent yarns adhere to each other, and the other side can act as a compression spring to elastically push up the inside of the skin layer.

For example, as shown in FIG. 20, when the lifting thread 30 according to the third example embodiment of the present disclosure is inserted into the nasal cavity (nose) of a human body, the compression spring portion (upper portion in the drawing) of the lifting thread 30 according to the third example embodiment of the present disclosure may elastically push up the tip of the nose to maintain a straight nose, while the tension spring portion (lower portion in the drawing) is in close contact with each other like a pillar (base) to firmly support the entire lifting thread 30, thereby enabling a stable procedure.

Furthermore, the method for manufacturing the lifting threads according to the first to third example embodiments of the present disclosure may further include a heat treatment (thermoforming process) to improve the elasticity of the medical thread. The heat treatment step may heat-treat the medical thread (yarn) wound around the needle so that, even when the lifting thread is pulled out of the needle and separated, the lifting thread maintains a tensile coil spring or compressed coil spring shape, and further improves the elasticity of the lifting thread.

As described above, in the present disclosure, since the medical thread (yarn) is pre-twisted (twisted) to increase elasticity, there is no need to increase the heat treatment temperature to a high temperature (a temperature exceeding 75% of the melting temperature, for example, over 85°C in the case of polydioxanone polymer) during the heat treatment process, and sufficient elasticity may be achieved even with heat treatment at a low temperature (a low temperature of 45% or less of the melting temperature - a low temperature of 50°C or less in the case of polydioxanone) so not only may the manufacturing cost of the lifting thread be reduced, but also the yield may be increased, and the lifting thread has the advantage of maximizing the effect of the procedure because the time until hydrolysis is sufficiently long after the procedure is performed to embed the thread in the human body.

Meanwhile, FIG. 21 is a view showing the lifting thread 10 according to the first example embodiment of the present disclosure.

Referring further to FIG. 21, the lifting thread 10 according to the first example embodiment of the present disclosure may be formed to have tensile coil spring characteristics by twisting the medical thread (yarn) in one direction so that the torsional stress is generated inside the medical thread and winding the medical thread (yarn) from the tip of the outer peripheral surface of the needle 1 toward the rear end portion thereof in the same direction as the twisting direction of the medical thread (yarn) so that, during the skin lifting procedure, when the lifting thread 10 is tensioned, a first elastic force according to the torsional stress and a second elastic force that is the combination of the spring tensile stress are applied.

That is, since the lifting thread in the related art does not have a twisted structure and thus does not generate torsional stress, only tensile stress is generated when the lifting thread is tensioned (pulled to both sides) during the skin lifting procedure, but the lifting thread 10 according to the first example embodiment of the present disclosure is configured to have tensile coil spring characteristics by first twisting the medical thread in one direction and then winding (coiling) in the same direction as the twisting direction of the medical thread so that when the lifting thread is tensioned during the skin lifting procedure, the first elastic force according to the torsional stress and the second elastic force that is the combination of the spring tensile stress act, thereby generating an effect of generating a stronger elastic restoring force compared to a general spring-type lifting thread in which the medical thread (yarn) is not twisted in advance.

Normally, lifting threads go through a process of increasing elasticity through heat treatment. However, when the heat treatment temperature of the lifting thread is too high (exceeding 75% of the melting temperature, for example, exceeding 85°C in the case of polydioxanone polymer), the elasticity of the lifting thread is improved, but during the skin lifting procedure, the lifting thread embedded in the skin layer takes too short a time for hydrolysis, and thus it is easily hydrolyzed, resulting in a disadvantage of shortening the duration of the treatment effect.

However, since the lifting thread 10 according to the first example embodiment of the present disclosure may be manufactured in a high-elasticity state by subjecting a medical thread to first twisting in one direction and then winding (coiling) it in the same direction as the twisting direction of the medical thread to have tensile coil spring characteristics, it is not necessary to treat the heat treatment temperature of the lifting thread 10 at a high temperature (a temperature exceeding 75% of the melting temperature, for example, exceeding 85°C in the case of polydioxanone polymer) more than necessary in the heat treatment of the lifting thread 10 so that in the skin lifting procedure as described above, the time for hydrolysis of the lifting thread 10 embedded in the skin layer is shortened so that the disadvantage of being easily hydrolyzed and thus shortening the treatment effect period may be fundamentally resolved.

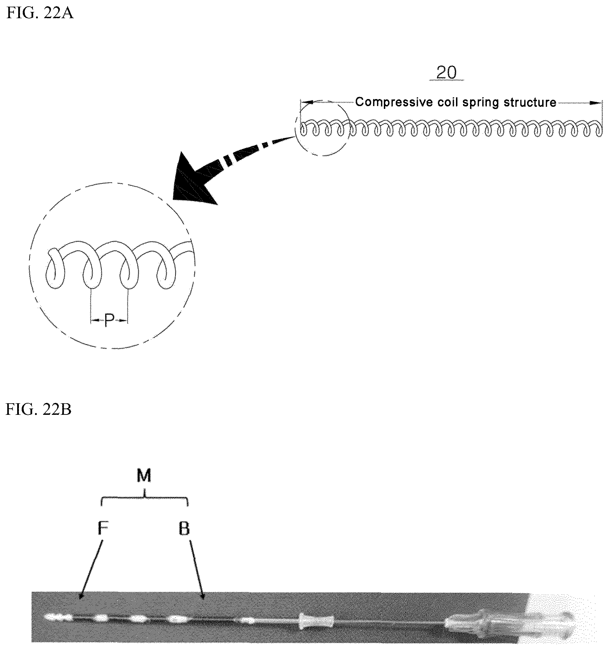

Also, FIG. 22a is a view showing the lifting thread 20 according to the second example embodiment of the present disclosure.

Referring further to FIG. 22a, the lifting thread 20 according to the second example embodiment of the present disclosure may be formed to have compressive coil spring characteristics by twisting the medical thread in one direction so that the torsional stress is generated inside the medical thread and winding the medical thread (yarn) from the tip of the outer peripheral surface of the needle 1 toward the rear end portion thereof in the direction oppsite to the twisting direction of the medical thread so that during the skin lifting procedure, when the lifting thread 20 is compressed, a first elastic force according to the torsional stress and a second elastic force that is the combined compressive stress are applied.

That is, since the lifting thread in the related art does not have a twisted structure, only compressive stress is generated when the lifting yarn is compressed (pressed from both sides) during the skin lifting procedure, but the lifting thread 20 according to the second example embodiment of the present disclosure may be configured to have compressive coil spring characteristics by subjecting the medical thread to first twisting in one direction and then winding (coiling) it in the direction opposite to the twisting direction of the medical thread so that, when the lifting thread is compressed during the skin lifting procedure, the first elastic force according to the torsional stress and the second elastic force that is the combined spring compressive stress act, thereby generating a stronger elastic restoring force compared to a general spring-type lifting thread that does not twist the medical thread (yarn) in advance.

Normally, lifting threads go through a process of increasing elasticity through heat treatment. However, when the heat treatment temperature of the lifting thread is too high (exceeding 75% of the melting temperature, for example, exceeding 85°C in the case of polydioxanone polymer), the elasticity of the lifting thread is improved, but during the skin lifting procedure, the lifting thread embedded inside the skin layer takes too short a time for hydrolysis, and thus it is easily hydrolyzed, resulting in a disadvantage of shortening the duration of the treatment effect.

However, since the lifting thread 20 according to the second example embodiment of the present disclosure is manufactured in a high-elasticity state by twisting a medical thread in one direction and then winding (coiling) it in the direction oppsite to the twisting direction of the medical thread to have compressive coil spring characteristics, it is not necessary to treat the heat treatment temperature of the lifting thread 20 at a high temperature (a temperature exceeding 75% of the melting temperature, for example, exceeding 85°C in the case of polydioxanone polymer) more than necessary in the heat treatment of the lifting thread 20 so that in the skin lifting procedure as described above, the time for hydrolysis of the lifting thread 20 embedded inside the skin layer is shortened so that the disadvantage of being easily hydrolyzed and thus shortening the duration of the treatment effect can be fundamentally resolved.

FIG. 22b is a photograph showing the multi-stage lifting thread according to the first and/or second example embodiment of the present disclosure.

Referring to FIG. 22b, the lifting thread according to the first and/or second example embodiment described above may be configured to form a multi-stage lifting thread M by connecting a plurality of lifting threads in series. The multi-stage lifting thread (M) may be a plurality of independent lifting threads that are the same or different from each other connected in series, and specifically, the multi-stage lifting thread M may be a number of n independent lifting threads that are the same or different from each other connected in series through n-1 connecting portions (not shown) (wherein n is any positive integer). The multi-stage lifting thread M may have a higher elastic modulus of the lifting thread F located at the front end with respect to the entry direction of the lifting thread during the lifting procedure than the lifting thread B located at the rear end. For example, the elastic modulus of the lifting thread F at the front end, for example, the first lifting thread F, may be 1/10 or more greater than the elastic modulus of the lifting thread B at the rear end, for example, the nth lifting thread B. Therefore, by using the multi-stage lifting thread M by connecting a plurality of lifting threads in series during the lifting procedure, the lifting thread F at the front end of the lifting thread M may exhibit an effect of better withstanding the frictional force against the skin tissue than when using a single type of lifting thread (lifting thread according to the first and/or second example embodiment described above). At this time, the connecting portions connecting the adjacent lifting threads may be connected through anchors at the ends of the lifting threads, or may also be connected by various methods such as fusion or bonding.

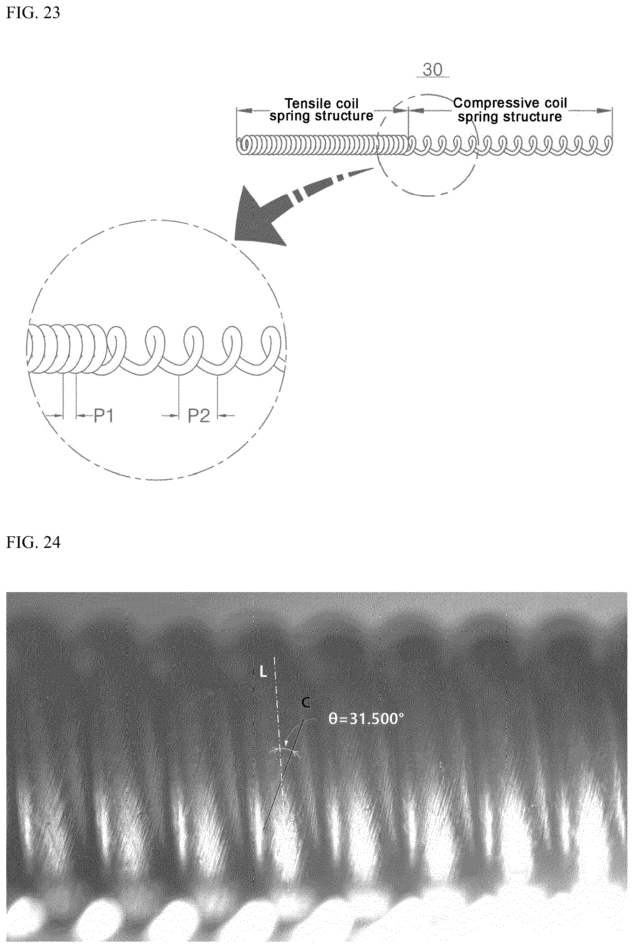

Also, FIG. 23 is a view showing the lifting thread 30 according to the third example embodiment of the present disclosure.

Referring further to FIG. 23, the lifting thread 30 according to the third example embodiment of the present disclosure may be formed to have tensile coil spring characteristics by first twisting the medical thread in one direction so that the torsional stress is generated inside the medical thread, and then first winding one end of the medical thread in the same direction as the first twisting direction from the tip of the outer peripheral surface of the needle 1 toward the rear end portion thereof. Afterwards, the remaining portion of the medical thread is twisted a second time in the direction opposite to the first twisting direction so that the torsional stress is generated in the opposite direction inside the medical thread, and the remaining portion of the medical thread is wound a second time in the same direction as the first twisting direction toward the rear end portion on the outer peripheral surface of the needle 1 so as to have compressive coil spring characteristics, thereby forming a composite structure having tensile coil spring characteristics and compressive coil spring characteristics.

The lifting thread 30 according to the third example embodiment of the present disclosure may be configured so that, during a lifting procedure, the tension coil spring portion of the lifting thread 30 in the skin layer has the dense pitch P1 to act as a pillar, and the compression coil spring portion has the non-dense pitch P2 to act as a pushing up of the skin layer.

The elasticity according to the torsional stress of the lifting thread 30 may be configured to be adjusted according to changes in the twist angle of the medical thread, the thickness of the medical thread, the inner diameter of the lifting thread, the pitch, or a combination thereof.

The medical threads may typically be made of threads with a diameter of 0.15 to 0.8 mm, such as silicone, polypropylene, polydioxanone, and poly(L-lactic acid) (PLLA). According to the present disclosure, the medical threads are not limited to threads, and all other materials using medical materials are also possible.

As explained above, the present disclosure has the following effects.

First, by twisting a medical thread and then winding it around the outer peripheral surface (circumference) of a needle to manufacture a lifting thread in a coil spring shape, when the lifting thread is tensioned or compressed during a skin lifting procedure, a first elastic force according to the torsional stress generated by twisting the medical thread and a second elastic force that is the combined tensile or compressive stress generated when the lifting thread is tensioned or compressed during the skin lifting procedure are applied so that, even after a long time has passed after the skin lifting procedure, a significant amount of elasticity remains so that sagging of the lifted skin does not occur at all and the elasticity of the skin is maintained as it is, thereby maximizing the lifting effect.

Second, normally, the lifting thread goes through a process of increasing elasticity through heat treatment. However, when the heat treatment temperature of the lifting thread is too high as in the past (exceeding 75% of the melting temperature, for example, exceeding 85°C in the case of polydioxanone polymer), the elasticity of the lifting thread is improved, but during the skin lifting procedure, the lifting thread embedded inside the skin layer takes too short a time for hydrolysis, and thus it is easily hydrolyzed and the duration of the procedure effect is shortened. However, the lifting thread of the present disclosure is manufactured in a state of high elasticity by subjecting the medical thread to first twisting in one direction and then winding (coiling) it in the same direction or opposite to the twisting direction of the medical thread to have tensile or compressive coil spring characteristics. Therefore, there is no need to treat the heat treatment temperature of the lifting thread to a high temperature (exceeding 75% of the melting temperature, for example, exceeding 85°C in the case of polydioxanone polymer) more than necessary during the heat treatment of the lifting thread. Thus, during the skin lifting procedure, the time for hydrolysis of the lifting thread embedded in the skin layer is shortened. Therefore, the disadvantage of being easily hydrolyzed and thus shortening the duration of the treatment effect can be fundamentally resolved.

Third, when the lifting thread of the composite coil spring structure is applied to the nasal cavity (nose) of the human body, the compression spring part of the lifting thread pushes up the tip of the nose to maintain a straight nose, while the tension spring part is tightly attached to each other like a pillar (base) to firmly support the entire lifting thread, enabling a stable procedure.

Fourth, since the procedure can be performed in a stable state due to the role of the pillar above, there is an effect that the procedure can be performed safely without the lifting thread being bent due to carelessness in the procedure, causing plastic deformation (permanent deformation) and loss of elastic restoring force.

Fifth, when embedding the lifting thread inside the skin layer, the lifting thread is supported by the non-slip joint and is smoothly inserted inside the skin layer without being pushed back. In addition, when the tip of the lifting thread is fixed while the lifting thread is embedded inside the skin layer and the needle is pulled out from the skin layer, the lifting thread stretches and generates elastic restoring force, making the procedure very convenient.

Meanwhile, although sthe present specification and drawings disclose preferred embodiments of the present disclosure, and pecific terms are used, they are used only in a general sense to easily describe the technical contents of the present disclosure and help understand the invention, and are not intended to limit the scope of the present disclosure. It will be apparent to a person skilled in the art to which the present disclosure pertains that other modified examples based on the technical idea of the present disclosure are possible in addition to the embodiments disclosed herein.

For example, accessories such as an anchor, a hook jaw, a withdrawal portion, and a non-slip combination portion are not limited to the lifting thread insertion device 100 according to the first example embodiment of the present disclosure, and can be equally applied to other example embodiments. In addition, the technique of the present disclosure is not limited to the skin lifting procedure, and can be equally applied to plastic surgery or other procedures.

Example 1: Lifting thread of tensile coil spring structure with torsional rotation of approximately 480

A 1 m medical thread was twisted approximately 480 times to produce a twisted thread having a twist per meter (TPM) of approximately 480. This twisted thread was coiled to produce a lifting thread having a tensile coil spring structure. Specifically, the twisted thread was positioned on a counterclockwise rotating tubular needle circumference, and the twisted thread was wound in a direction from the tip of the needle toward the rear end portion. The inner diameter of the coil spring is the same as the outer diameter of the needle. At this time, the lifting thread of the tensile coil spring structure produced has a Z-twist shape, specifically, the winding direction of the element wire is left-hand, that is, when the spring is lying parallel to the ground when looking at the winding direction of the element wire from the observer, the direction is from upper left to lower right.

FIG. 24 is a surface photograph of the lifting thread of the tensile coil spring structure according to Example 1 of the present disclosure.

Referring to FIG. 24, it can be confirmed that the lifting thread of the tensile coil spring structure, which is manufactured by twisting so that the number of torsional turns is approximately 480, has a twist angle $\theta$ of approximately 31.500°.

Example 2: Lifting thread of tensile coil spring structure with torsional rotation of approximately 420

A lifting thread having a tensile coil spring structure was manufactured using the same method as Example 1, except that a 1 m medical thread was twisted approximately 420 times to manufacture a twisted thread having a torsional rotation of approximately 420.

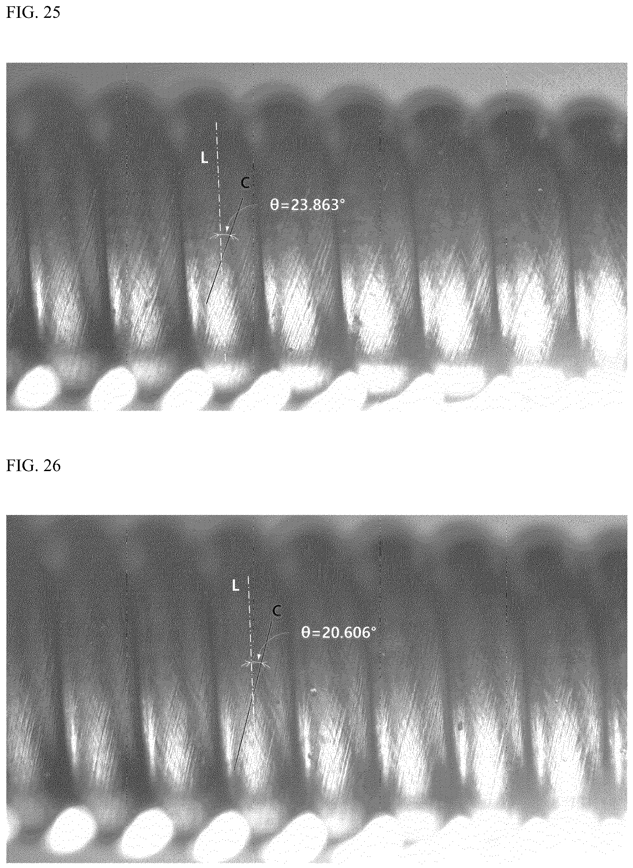

FIG. 25 is a surface photograph of the lifting thread of the tensile coil spring structure according to Example 2 of the present disclosure.

Referring to FIG. 25, it can be confirmed that the lifting thread of the tensile coil spring structure, which is manufactured by twisting so that the number of torsional turns is approximately 420, has a twist angle $\theta$ of approximately 23.863°.

Example 3: Lifting thread of a tensile coil spring structure with a torsional rotation of approximately 360

A lifting thread having a tensile coil spring structure was manufactured using the same method as Example 1, except that a 1 m medical thread was twisted approximately 360 times to manufacture a twisted thread having a torsional rotation of approximately 360.

FIG. 26 is a surface photograph of the lifting thread of the tensile coil spring structure according to Example 3 of the present disclosure.

Referring to FIG. 26, the lifting thread of the tensile coil spring structure, manufactured by twisting approximately 360 turns, has a twist angle θ of approximately 20.606°.

Example 4: Lifting thread of tensile coil spring structure with torsional rotation of approximately 300

A lifting thread having a tensile coil spring structure was manufactured using the same method as Example 1, except that a 1 m medical thread was twisted approximately 300 times to manufacture a twisted thread having a torsional rotation of approximately 300.

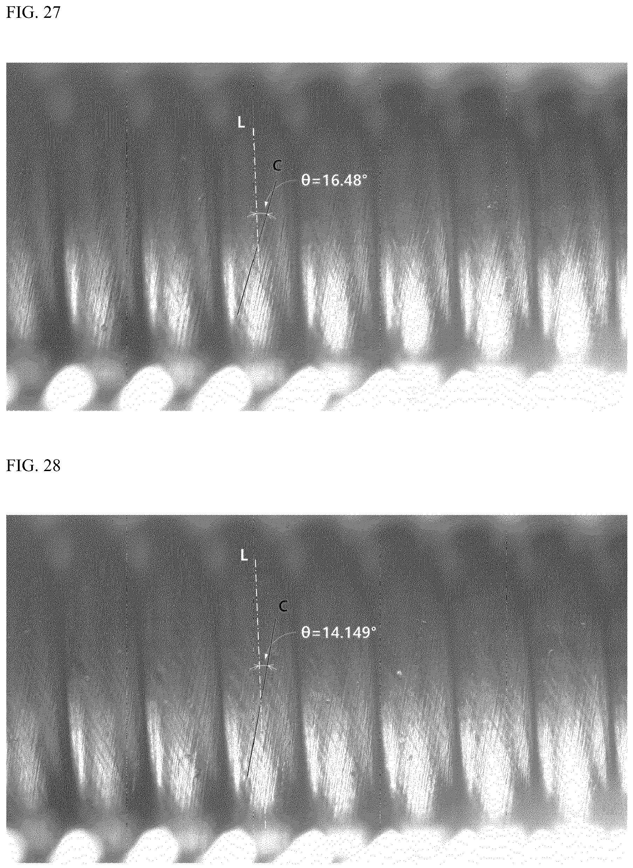

FIG. 27 is a surface photograph of the lifting thread of the tensile coil spring structure according to Example 4 of the present disclosure.

Referring to FIG. 27, the lifting thread of the tensile coil spring structure, manufactured by twisting approximately 300 turns, has a twist angle θ of approximately 16.48°.

Example 5: Lifting thread of tensile coil spring structure with torsional rotation of approximately 240

A lifting thread having a tensile coil spring structure was manufactured using the same method as Example 1, except that a 1 m medical thread was twisted approximately 240 times to manufacture a twisted thread having a torsional rotation of approximately 240.

FIG. 28 is a surface photograph of the lifting thread of the tensile coil spring structure according to Example 5 of the present disclosure.

Referring to FIG. 28, the lifting thread of the tensile coil spring structure, manufactured by twisting approximately 240 turns, has a twist angle θ of approximately 14.149°.

Example 6: Lifting thread of tensile coil spring structure with torsional rotation of approximately 180

A lifting thread having a tensile coil spring structure was manufactured using the same method as Example 1, except that a 1 m medical thread was twisted approximately 180 times to manufacture a twisted thread having a torsional rotation of approximately 180.

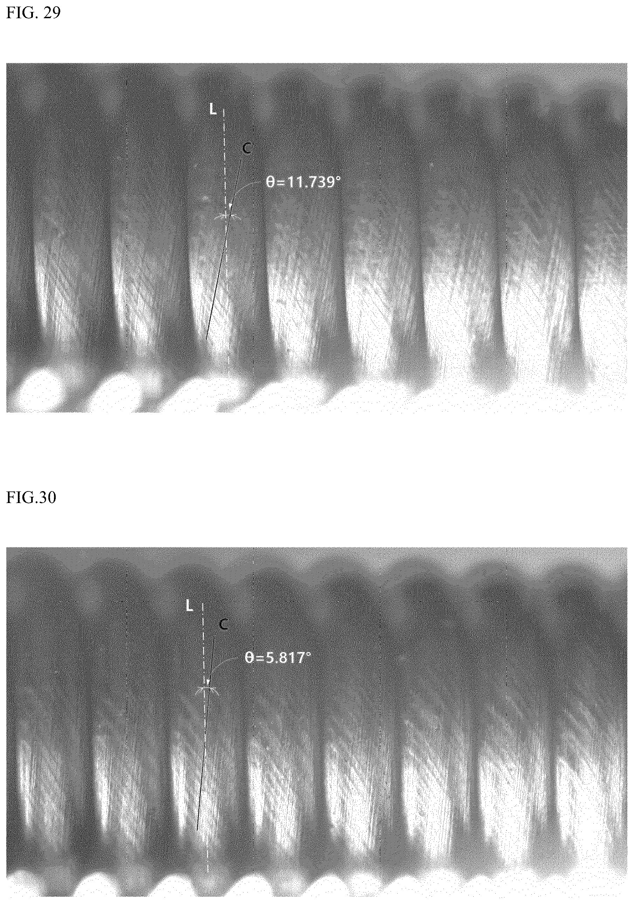

FIG. 29 is a surface photograph of the lifting thread of the tensile coil spring structure according to Example 6 of the present disclosure.

Referring to FIG. 29, the lifting thread of the tensile coil spring structure, manufactured by coiling a twisted thread twisted approximately 180 times, has a twist angle θ of approximately 11.793°.

Example 7: Lifting thread of tensile coil spring structure with torsional rotation of approximately 120

A lifting thread having a tensile coil spring structure was manufactured using the same method as Example 1, except that a 1 m medical thread was twisted approximately 120 times to manufacture a twisted thread having a torsional rotation of approximately 120.

FIG. 30 is a surface photograph of the lifting thread of the tensile coil spring structure according to Example 7 of the present disclosure.

Referring to FIG. 30, the lifting thread of the tensile coil spring structure, manufactured by coiling a twisted thread twisted approximately 120 times, has a twist angle θ of approximately 5.817°.

Example 8: Lifting thread of tensile coil spring structure with torsional rotation of approximately 60

A lifting thread having a tensile coil spring structure was manufactured using the same method as Example 1, except that a 1 m medical thread was twisted approximately 60 times to manufacture a twisted thread having a torsional rotation of approximately 60.

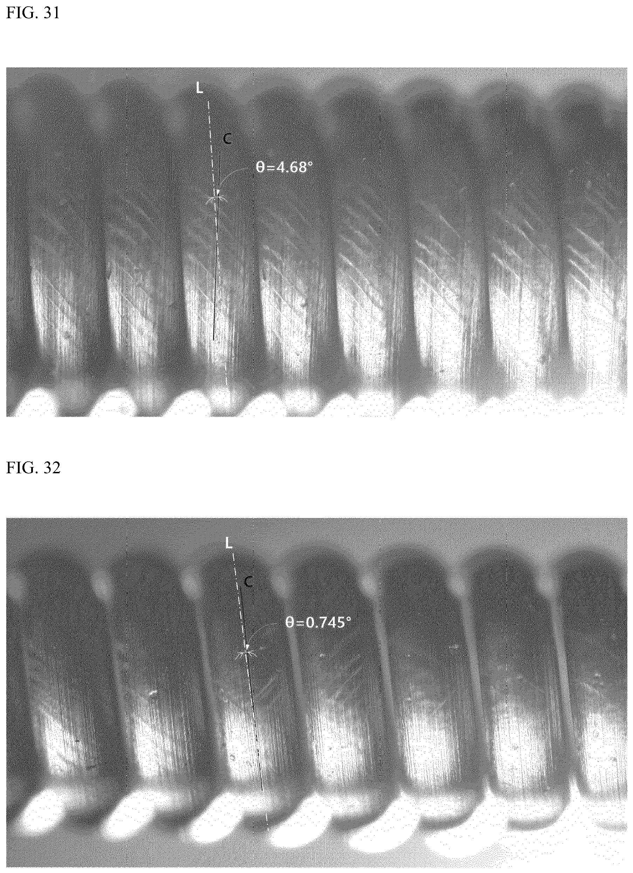

FIG. 31 is a surface photograph of the lifting thread of the tensile coil spring structure according to Example 8 of the present disclosure.

Referring to FIG. 31, the lifting thread of the tensile coil spring structure, manufactured by coiling a twisted thread twisted approximately 60 times, has a twist angle θ of approximately 4.68°.

Comparative Example 1: Lifting thread of tensile coil spring structure using untwisted medical thread

A 1 m medical thread was used without twisting and coiled to manufacture a lifting thread having a tensile coil spring structure.

FIG. 32 is a surface photograph of the lifting thread of the tensile coil spring structure according to Comparative Example 1 of the present disclosure.

Referring to FIG. 32, the lifting thread of the tensile coil spring structure, manufactured by coiling untwisted medical thread without using a twisted thread, has a twist angle θ of approximately 0.745°.

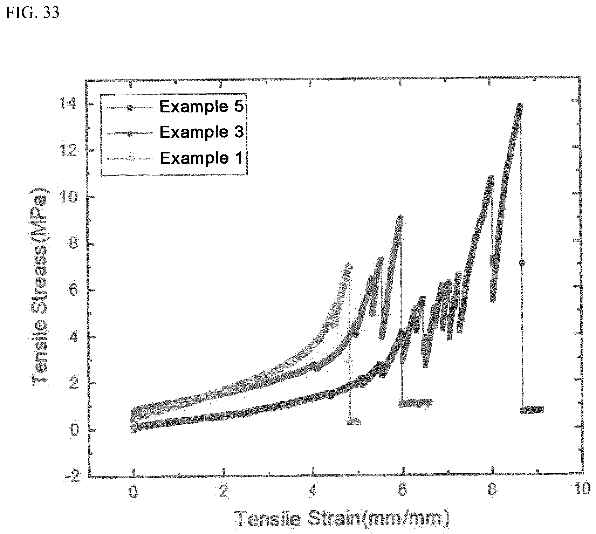

FIG. 33 is a graph showing the tensile stress as a function of tensile strain for lifting threads according to Examples 1, 3, and 5 of the present disclosure.

Referring to FIG. 33, it can be seen that the elastic modulus of lifting threads according to Examples 1, 3, and 5 of the present disclosure increases in order of torsional rotation.

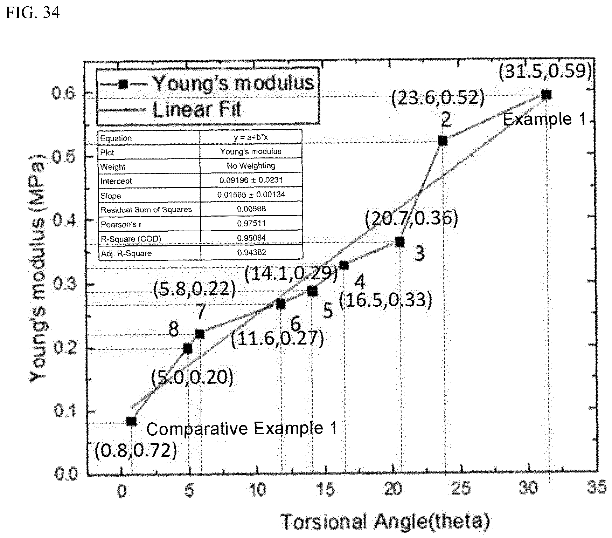

FIG. 34 is a graph showing the Young's modulus according to the reverse twist angle of the lifting thread according to Examples 1 to 8 and Comparative Example 1 of the present disclosure.

Referring to FIG. 34, Examples 1 to 8 of the present disclosure and Comparative Example 1 are about a lifting thread of a tensile spring structure, and when the Young's modulus (elastic modulus) was measured according to the reverse twist angle using the same, it was confirmed that the Young's modulus (elastic modulus) tends to increase linearly as the twist angle increases. The Young's modulus of Examples 1 to 8 of the present disclosure and Comparative Example 1 can be confirmed to be measured as 31.5 MPa, 23.6 MPa, 20.7 MPa, 16.5 MPa, 14.1 MPa, 11.6 MPa, 5.8 MPa, 5.0 MPa, and 0.8 MPa, respectively, and the twist angles at this time are 0.59, 0.52, 0.36, 0.33, 0.29, 0.27, 0.22, 0.20, and 0.72, respectively.

The specific explanation for deriving this is as follows.

When a load F is applied to a spring, a torsional deformation occurs in the medical thread corresponding to the element wire of the spring, and the torque T applied at this time can be expressed by Equation 1 below. The shear stress within the element wire of the spring can be derived through Equations 1 to 3 below.

[Equation 1]

$$T=F\left(\frac{D}{2}\right)$$

[Equation 2]

$$\tau=\frac{T\gamma}{2I}$$

[Equation 3]

$$\gamma=\frac{2sr}{\pi D^2}$$

In Equations 1 to 3, T is a torque applied when torsional deformation occurs in the spring element wire, F is a force applied in the axial direction, D is a coil diameter of the coil spring, I is a second moment of area of bending, $\gamma$ is a shear strain, r is a diameter of the element wire, and s is a change in extension of the coil along one rotational axis.

The elastic modulus k of a coil spring that increases the axial tensile deformation resistance of the coil spring by preemptively twisting the spring element wire may be expressed by Equation 4 below.

[Equation 4]

$$k=\frac{G(\theta)d^4}{8D^3N}$$

In Equation 4, G represents a shear modulus, which is a function of the twist angle $\theta$ of the spring element wire, d is a diameter of the element wire, D is a coil diameter of the coil spring, and N is the number of turns of the coil spring.

In summary, in the present disclosure, by forming a preset twist of the element wire in the opposite direction of the shear deformation (torsion) that occurs when the coil spring is tensioned, resistance to shear deformation is created, and by using this, the spring's axial tensile deformation resistance is increased, thereby increasing the spring's elastic modulus. Therefore, the more the above-described preset twist is formed (the larger the twist angle), the more resistance to shear deformation G is created, and the more the spring's axial tensile deformation resistance is increased, thereby increasing the elastic modulus k of the prsing. In summary, the above-described equations can be defined as a linear relationship between the shear elastic modulus G and the elastic modulus k of the spring.

Meanwhile, the embodiments of the present disclosure disclosed in this specification and drawings are merely specific examples presented to aid understanding and are not intended to limit the scope of the present disclosure. It will be apparent to those skilled in the art to which the present disclosure pertains that other modified examples based on the technical concepts of the present disclosure are also possible, in addition to the embodiments disclosed herein.

[Reference Signs List]

1: Needle
1a: Handle portion
10: Lifting thread according to first example embodiment of present disclosure
20: Lifting thread according to second example embodiment of present disclosure
30: Lifting thread according to third example embodiment of present disclosure
50, 500, 5000, 60: Anchor
51, 501, 5001: Fastening member
52, 502, 5002: Anchor body
70: Sliding holder
71: Grip portion
72: Sliding tube
80: Withdrawal portion
90: Non-slip combination portion
91a: Knot portion91b: Binding thread
100: Lifting thread insertion device according to first example embodiment of present disclosure
200: Lifting thread insertion device according to second example embodiment of present disclosure
300: Lifting thread insertion device according to third example embodiment of present disclosure
51a, 501a, 5001a: Spiral groove

51b, 501b, 5001b, 61b: Hook jaw
62: Temporary knot
63: Thread for temporary knot
P: Pitch
L: Longitudinal central axis (imaginary line) of twisted thread
C: Twist line deforming by twisting parallel line appearing in longitudinal direction of medical thread
M: Multi-stage lifting thread
F: First lifting thread at tip
B: Nth lifting thread at rear end portion

## Claims

**1.** A lifting thread of a coil spring structure in which a twisted thread formed by twisting one or more medical threads centered on a longitudinal central axis (L) of the medical thread is wound (coiled), wherein the lifting thread adjusts the elastic modulus of the lifting thread by adjusting a twist angle (θ), which is an angle at which the twisted thread is twisted.

**2.** A composite structure lifting thread (30) including a composite structure in which a first coil spring structure in which a first twisted thread in which a portion of one or more medical threads is twisted is wound (coiled) and a second coil spring structure in which a second twisted thread in which another part of the medical thread is twisted in an opposite direction is wound (coiled) are connected so that they operate independently, wherein
in the composite structure lifting thread (30), a first twist angle, which is an angle at which the first twisted thread is twisted, and a second twist angle, which is an angle at which the second twisted thread is twisted, are each independently adjusted to adjust the elastic modulus of the composite structure lifting thread (30).

**3.** The lifting thread of claim 1 or 2, wherein, as the twist angle (θ) increases, the elastic modulus of the lifting thread increases.

**4.** The lifting thread of claim 1 or 2, wherein the lifting thread is configured such that the elastic modulus of the lifting thread is adjusted by changing a thickness of the medical thread, an inner diameter of the lifting thread, a pitch, or a combination thereof.

**5.** The lifting thread of claim 1 or 2, wherein the lifting thread includes a multi-stage lifting thread (M) in which a plurality of independent lifting threads, which are the same or different, are connected in series and the multi-stage lifting thread (M) has a lifting thread (F) located at the front end with respect to an entry direction of the lifting thread during a lifting procedure, which has a higher elastic modulus than the other lifting thread (B) located at the rear end.

**6.** A method for manufacturing a lifting thread of a coil spring structure by winding a twisted thread formed by twisting a medical thread, the method comprising:

a twisted thread forming step of twisting the medical thread centered on a longitudinal central axis (L) of the medical thread so that a torsional stress is generated inside the medical thread; and
a lifting thread coiling step of forming a lifting thread by winding (coiling) the twisted thread into a coil spring shape,
wherein, during a skin lifting procedure, a first elastic force according to a torsional stress generated in the twisted thread forming step and a second elastic force, which is a combined elastic force generated by tension or compression during the skin lifting procedure, are applied.

**7.** A method for manufacturing a lifting thread (10) having tensile coil spring characteristics by winding a medical thread (1) on an outer peripheral surface of a needle (10), the method comprising:

a twisted thread forming step S110 of twisting the medical thread in one direction so that a torsional stress is generated inside the medical thread; and
a lifting thread coiling step S 120 of winding (coiling) the twisted thread from a tip of an outer peripheral surface of the needle toward a rear end portion thereof in the same direction as a twisting direction of the twisted thread to form the lifting thread (10) having tensile coil spring characteristics,
wherein, during a skin lifting procedure, the lifting thread (10) is configured to apply a first elastic force according to a torsional stress of the medical thread and a second elastic force that is a combination of a tensile stress resulting from tensioning the lifting thread (10).

**8.** A method for manufacturing a lifting thread (20) having compressive coil spring characteristics by winding a medical thread on an outer peripheral surface of a needle (1), the method comprising:

a twisted thread forming step (S210) of twisting the medical thread in one direction so that a torsional stress is generated inside the medical thread; and
a lifting thread coiling step (S220) of forming the lifting thread (20) having compressive coil spring characteristics by winding (coiling) the twisted thread from a tip of an outer peripheral surface of the needle toward a rear end portion thereof in a direction opposite to a twisting direction of the twisted thread,
wherein, during a skin lifting procedure, the lifting thread (20) is configured to apply a first elastic force according to a torsional stress of the medical thread and a second elastic force that is a combination of a compressive stress resulting from compressing the lifting thread (20).

**9.** A method for manufacturing a lifting thread (30) of a composite structure having tensile coil spring characteristics and compressive coil spring characteristics, the method comprising:

a first twisted thread forming step (S310) of twisting the medical thread in one direction so that a torsional stress is generated inside the medical thread;
a first coiling step (S320) of winding a first twisted thread into a coil spring shape from a tip of an outer peripheral surface of a needle (1) toward a rear end portion thereof;
a second twisted thread forming step (S330) of twisting the remaining portion of the medical thread in a direction opposite to a first twisting direction so that a torsional stress is generated in an opposite direction inside the medical thread; and
a second coiling step (S340) of winding a second twisted thread in the same direction as the first twisting direction toward a rear end portion of the outer peripheral surface of the needle (1).

**10.** The method of any one of claims 6 to 9, further comprising a heat treatment to improve elasticity of the lifting thread.

**11.** A lifting thread insertion device comprising:

a needle (1) used for medical; and
a lifting thread formed to have tensile coil spring characteristics by twisting a medical thread in one direction so that a torsional stress is generated inside the medical thread and winding the medical thread from a tip of an outer peripheral surface of the needle (1) toward a rear end portion thereof in the same direction as a twisting direction of the medical thread,
wherein, during a skin lifting procedure, a first elastic force according to a torsional stress generated by twisting the medical thread in advance and a second elastic force resulting from a tensile stress generated by tensioning a tensile coil spring are combined to act together.

**12.** A lifting thread insertion device comprising:

a needle (1) used for medical; and
a lifting thread formed to have compressive coil spring characteristics by twisting a medical thread in one direction so that a torsional stress is generated inside the medical thread and winding the medical thread from a tip of an outer peripheral surface of the needle (1) toward a rear end portion thereof in a direction opposite to a twisting direction of the medical thread,
wherein, during a skin lifting procedure, a first elastic force according to a torsional stress generated by twisting the medical thread in advance and a second elastic force that is a combined compressive stress generated by compressing a compression coil spring are applied.

**13.** A lifting thread insertion device comprising:

a needle (1) used for medical; and
a lifting thread in which a portion of a medical thread is first twisted in one direction so that a torsional stress is generated inside the medical thread, and is wound in a first coil spring shape from a tip of an outer peripheral surface of the needle (1) toward a rear end portion thereof, and the remaining portion of the medical thread is second twisted in a direction opposite to a first twisting direction, and is wound (coiled) in a second coil spring shape in the same direction as a first coil spring toward a rear end portion of the outer peripheral surface of the needle (1),
wherein the lifting thread includes tensile coil spring characteristics and compressive coil spring characteristics.

**14.** The lifting thread insertion device of any one of claims 11 to 13, wherein the lifting thread includes a multi-stage lifting thread (M) in which a plurality of independent lifting threads, which are the same or different, are connected in series, and the multi-stage lifting thread (M) has a lifting thread (F) located at a front end with respect to an entry direction of the lifting thread during the lifting procedure, which has a higher elastic modulus than another lifting thread (B) located at the rear end.

**15.** The lifting thread insertion device of any one of claims 11 to 13, comprising anchors (50 and 60) provided on a tip and rear end portion of the lifting thread, respectively,
wherein, when the anchors (50 and 60) are pulled in a direction facing each other, hook jaws (51b and 61b) of the anchors (50 and 60) are provided in a direction facing each other so as to be caught in a skin layer and continuously maintain a tension state of the lifting thread.

**16.** The lifting thread insertion device of claim 15, wherein a temporary knot (62) is further provided to prevent physical interference in a process of the lifting thread being embedded into the skin layer in the hook jaw (61b) included in the anchor (60) provided in a rear end portion of the lifting thread.

**17.** The lifting thread insertion device of any one of claims 11 to 13, wherein a sliding holder (70) configured to support the rear end portion of the lifting thread so that the lifting thread does not come out of the skin layer together with the needle (1) when the needle (1) is pulled out from the skin layer during the skin lifting procedure, but remains embedded in the skin layer; and a withdrawal portion (80) connected to the rear end portion of the lifting thread so that the lifting thread is able to be pulled and tensioned by being exposed to an outside of the skin layer by a predetermined length when the lifting thread is embedded in the skin layer.

**18.** The lifting thread insertion device of any one of claims 11 to 13, further comprising:

a non-slip combination portion (90) formed on the rear end portion of the lifting thread and relatively tightly combined with an outer peripheral surface of the needle (1) compared to other portions so that, when the needle (1) is inserted into the skin layer during the lifting procedure, the lifting thread is not pushed backwards, and when a location of a tip of the lifting thread is fixed in the skin layer and the needle (1) is pulled backwards, the rear end portion of the lifting thread is pulled out together with the needle (1) and is tensioned to have elastic restoring force; and

a sliding holder (70) that is formed in a pipe shape and is connected to the outer peripheral surface of the needle (1) so as to be able to slide forward and backward, and that is able to separate the needle (1) from the skin layer while supporting the rear end portion of the lifting thread when separating the lifting thread from the needle (1) during the lifting procedure.

**19.** The lifting thread insertion device of any one of claims 11 to 13, wherein elasticity of the lifting thread according to the torsional stress is adjusted by changing a twist angle of the medical thread, a thickness of the medical thread, an inner diameter of the lifting thread, a pitch, or a combination thereof.

**20.** The lifting thread insertion device of any one of claims 11 to 13, wherein, during the lifting procedure, the tension coil spring portion of the lifting thread in the skin layer has a dense pitch (P1) to act as a pillar, and the compression coil spring portion has a non-dense pitch (P2) to act as a lifting layer.

FIG. 1

9
6
11

FIG. 2

100
10
1a

FIG. 3

10
P

FIG. 4

51b
50
10
51b
10
61b
50
60
1a

FIG. 5

10
61b
60
50
51b
Skin layer

FIG. 6

50
51
51b
52
10
35°
51a
51
52
10
50

FIG. 7

500
501
502
10
35°
501a
501b
501
502
10
500

FIG. 8

5000
5002
5001 5001b
10
35°
10
5001a
5000
5002
5001 5001b
10
35°
5001a
5002
5001
10
5000

FIG. 9

10
61b
60
63
62

FIG. 10

100
1
70
71
72
1a
80
10
1
10
80
71

FIG. 11

100
70
1a
71
72
10
10
1
90
1
71

FIG. 12

100
70
71
72
1a
10
1
10
91
71
91a
91b

FIG. 13

100
20
1
70
71
72
1a

FIG. 14

20
P

FIG. 15

100
30
70
1
71
72
1a

FIG. 16

Tensile coil spring structure
Compressive coil spring structure
P1
P2

FIG. 17

Twisting thread forming step (S110) of twisting medical thread in one direction so that torsional stress is generated inside medical thread

Lifting thread coiling step (S120) of forming lifting thread having tensile coil spring characteristics by winding (coiling) twisted thread from tip of outer surface of needle toward rear end portion thereof in same direction as twisting direction of twisted thread

Heat treatment

FIG. 18

Twisting thread forming step (S210) of twisting medical thread in one direction so that torsional stress is generated inside medical thread.

Lifting thread coiling step (S220) of forming lifting thread having compression coil thread characteristics by winding (coiling) twisted thread from tip of outer surface of needle toward rear end portion thereof in direction opposite to twisting direction of twisted thread

Heat treatment

FIG. 19

First twisting thread forming step (S310) of twisting medical thread in one direction so that torsional stress is generated inside medical thread

↓

First coiling step (S320) of winding first twisting thread in coil spring shape from tip of outer surface of needle toward rear end portion thereof

↓

Second twisting thread forming step (S330) of twisting remaining portion of medical thread in direction opposite to first twisting direction so that twisting stress is generated in opposite direction inside medical thread

↓

Second coiling step (S340) of winding second twisting thread around outer surface of needle toward rear end portion in same direction as first coiling direction.

FIG. 20

Push-up
Nasal cavity(nose)
30
Pilar(base)

FIG. 21

10
Tensile coil spring structure

FIG. 22A

20
Compressive coil spring structure
P

FIG. 22B

M
F
B

FIG. 23

30
Tensile coil spring structure
Compressive coil spring structure
P1
P2

FIG. 24

L
C
θ=31.500°

FIG. 25

L
C
θ=23.863°

FIG. 26

L
C
θ=20.606°

FIG. 27

L
C
θ=16.48°

FIG. 28

L
C
θ=14.149°

FIG. 29

L
C
θ=11.739°

FIG.30

L
C
θ=5.817°

FIG. 31

L
C
θ=4.68°

FIG. 32

L
C
θ=0.745°

FIG. 33

Example 5
Example 3
Example 1
Tensile Streass(MPa)
Tensile Strain(mm/mm)
14
12
10
8
6
4
2
0
-2
0
2
4
6
8
10

FIG. 34

Young's modulus
Linear Fit
Equation | y = a+b*x
Plot | Young's modulus
Weight | No Weighting
Intercept | 0.09196 ± 0.0231
Slope | 0.01565 ± 0.00134
Residual Sum of Squares | 0.00988
Pearson's r | 0.97511
R-Square (COD) | 0.95084
Adj. R-Square | 0.94382
(31.5,0.59)
(23.6,0.52)
2
Example 1
(20.7,0.36)
3
(14.1,0.29)
4
(16.5,0.33)
5
(5.8,0.22)
7
8
6
(11.6,0.27)
(5.0,0.20)
Comparative Example 1
(0.8,0.72)
Young's modulus (MPa)
Torsional Angle(theta)
0.0
0.1
0.2
0.3
0.4
0.5
0.6
0
5
10
15
20
25
30
35

## INTERNATIONAL SEARCH REPORT

International application No. **PCT/KR2024/012552**

### A. CLASSIFICATION OF SUBJECT MATTER

**A61B 17/06**(2006.01)i; **A61B 17/34**(2006.01)i; **A61F 2/00**(2006.01)i; **A61B 17/00**(2006.01)i

According to International Patent Classification (IPC) or to both national classification and IPC

### B. FIELDS SEARCHED

Minimum documentation searched (classification system followed by classification symbols)

A61B 17/06(2006.01); A61B 17/00(2006.01); A61B 17/34(2006.01); A61F 2/00(2006.01); D02J 3/02(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 수술(surgical), 리프팅(lifting), 실(thread), 권취(winding), 코일(coil), 트위스트(twist)

### C. DOCUMENTS CONSIDERED TO BE RELEVANT

| Category* | Citation of document, with indication, where appropriate, of the relevant passages | Relevant to claim No. |
|---|---|---|
| Y | KR 10-1822640 B1 (PARK, Eun Hui) 26 January 2018 (2018-01-26) See paragraphs [0043]-[0045] and [0077]; and figures 2-6 and 14. | 1,3,4 |
| A | | 2,5-20 |
| Y | KR 10-2020-0145314 A (G-MEDIENCE CO., LTD.) 30 December 2020 (2020-12-30) See paragraphs [0071]-[0101]; and figure 2. | 1,3,4 |
| A | KR 10-2353171 B1 (KIM, Ki Won) 19 January 2022 (2022-01-19) See paragraph [0042]; and figures 1 and 2. | 1-20 |
| A | KR 10-1642962 B1 (JWORLD CO., LTD.) 26 July 2016 (2016-07-26) See paragraph [0067]; and figures 1 and 2. | 1-20 |
| A | KR 10-2016-0074933 A (JO, Hyon Chan et al.) 29 June 2016 (2016-06-29) See paragraph [0050]; and figures 2, 3 and 5. | 1-20 |

☐ Further documents are listed in the continuation of Box C.  ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **25 November 2024** | **25 November 2024** |

| Name and mailing address of the ISA/KR | Authorized officer |
|---|---|
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
**Information on patent family members**

International application No.
**PCT/KR2024/012552**

| Patent document cited in search report | Publication date (day/month/year) | Patent family member(s) | Publication date (day/month/year) |
|---|---|---|---|
| KR 10-1822640 B1 | 26 January 2018 | None | |
| KR 10-2020-0145314 A | 30 December 2020 | None | |
| KR 10-2353171 B1 | 19 January 2022 | None | |
| KR 10-1642962 B1 | 26 July 2016 | CN 108350619 A | 31 July 2018 |
| | | CN 108350619 B | 12 March 2021 |
| | | EP 3369848 A1 | 05 September 2018 |
| | | EP 3369848 A4 | 03 July 2019 |
| | | JP 2019-502502 A | 31 January 2019 |
| | | JP 6650046 B2 | 19 February 2020 |
| | | US 10918378 B2 | 16 February 2021 |
| | | US 11627957 B2 | 18 April 2023 |
| | | US 2018-0317911 A1 | 08 November 2018 |
| | | US 2021-0128148 A1 | 06 May 2021 |
| | | WO 2017-078492 A1 | 11 May 2017 |
| KR 10-2016-0074933 A | 29 June 2016 | KR 10-1748755 B1 | 19 June 2017 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1726317 A
- EP 04775207